# EUROPÄISCHE PATENTSCHRIFT

(11) **EP 2 694 917 B1**
(45) Veröffentlichungstag und Bekanntmachung des Hinweises auf die Patenterteilung: **23.11.2016**
(21) Anmeldenummer: 12718897.7
(22) Anmeldetag: 14.03.2012
(51) Int. Cl.: G01C 21/34, G01C 21/36, G06Q 10/10, A61B 5/0205, A61B 5/021, A61B 5/024, A61B 5/0402, A61B 5/08, A61B 5/18, A61B 5/145

(54) **VERFAHREN UND VORRICHTUNG ZUM DURCHFÜHREN EINER REISEROUTENPLANUNG FÜR EIN FAHRZEUG**
METHOD AND APPARATUS FOR CARRYING OUT TRAVEL ROUTE PLANNING FOR A VEHICLE
PROCÉDÉ ET DISPOSITIF POUR EFFECTUER UN CALCUL D'ITINÉRAIRE POUR UN VÉHICULE

(30) Priorität: 01.04.2011 DE 102011015775
(43) Veröffentlichungstag der Anmeldung: 12.02.2014
(73) Patentinhaber: Volkswagen Aktiengesellschaft, 38436 Wolfsburg (DE)
(72) Erfinder: HOCH, Nicklas, 30559 Hannover (DE); WERTHER, Bernd, 38170 Klein Vahlberg (DE)
(86) Internationale Anmeldenummer: PCT/EP2012/001127
(87) Internationale Veröffentlichungsnummer: WO 2012/130388

(56) Entgegenhaltungen:
- EP-A1- 1 909 069
- EP-A1- 1 944 724
- EP-A2- 1 975 561
- EP-A2- 1 975 562
- US-A- 5 815 824

## Beschreibung

Die vorliegende Erfindung betrifft ein Verfahren und eine Vorrichtung zum Durchführen einer Reiseroutenplanung für ein Fahrzeug. Das Fahrzeug umfasst einen Energiespeicher zum Speichern der Energie zum Antrieb des Fahrzeugs, insbesondere eine wiederaufladbare Batterie. Ergänzend oder alternativ kann auch ein herkömmlicher Antrieb mittels eines Kraftstoffes vorgesehen sein. Bei dem Fahrzeug handelt es sich somit insbesondere um ein sogenanntes Elektrofahrzeug oder ein sogenanntes Hybridfahrzeug.

Für den Nutzer eines Fahrzeugs stellt sich häufig das Problem, dass er innerhalb eines bestimmten Zeitraums verschiedene Termine an verschiedenen Orten wahrnehmen muss. Hierfür soll eine Reiseroutenplanung für das Fahrzeug durchgeführt werden. Die Reiseroute soll dabei so geplant werden, dass der Nutzer mit dem Fahrzeug die Orte, bei denen die Termine stattfinden, rechtzeitig erreicht. Dabei ist zu berücksichtigen, dass von Zeit zu Zeit der Energiespeicher wieder aufgefüllt werden muss. Insbesondere bei Elektrofahrzeugen besteht ein Bedürfnis, das Wiederaufladen der Batterie möglichst gut in die Reiseroutenplanung einzubeziehen, da üblicherweise die Reichweite eines Elektrofahrzeugs wesentlich geringer ist als die Reichweite eines herkömmlichen Fahrzeugs, welches mittels eines Kraftstoffs angetrieben wird.

Aus der DE 195 19 107 C1 ist eine Fahrtroutenratgebereinrichtung insbesondere für ein Elektrofahrzeug bekannt. Die beschriebene Einrichtung umfasst eine Dateneingabeeinheit zur Eingabe eines oder mehrerer Zielorte für eine Fahrt und einen Wegenetzspeicher zur Abspeicherung der auf dem vom Fahrzeug befahrbaren Wegenetz liegenden Orte und der zugehörigen Ortsentfernungen. Ferner umfasst die Einrichtung eine Rechnereinheit zur Bestimmung einer oder mehrerer möglicher Fahrtrouten vom Fahrzeugstandort zu den Zielorten einschließlich erforderlicher Energieeinspeisevorgänge an einem oder mehreren Energieeinspeiseorten in Abhängigkeit von der im Energiespeicher vorhandenen Energiemenge, dem Energieeinspeisenetz und dem streckenspezifischen Energieverbrauch. Schließlich umfasst die Einrichtung eine Anzeigeeinheit zur Anzeige der von der Rechnereinheit bestimmten Fahrtrouten.

In der DE 10 2004 022 265 A1 ist ein Verfahren zum Ausarbeiten einer Route von einem Ausgangspunkt zu einem Zielpunkt in einem Navigationssystem beschrieben. Bei dem Verfahren wird bei der Ausarbeitung der Route ein Faktor, der den Energieverbrauch zum Zurücklegen der Route beeinflusst, berücksichtigt.

Aus der DE 100 59 746 A1 ist ein Verfahren zur computerunterstützten Reiseroutenplanung und Reiseroutenführung bekannt, welches dynamische Änderungen der Verkehrssituation und Änderungen von Terminen berücksichtigt.

Aus der EP 1 300 817 B1 ist ein Navigationsdaten-Bereitstellungssystem bekannt. Bei diesem System werden Fahrtroutendaten ausgesandt, die in Abhängigkeit von Nutzerpräferenzdaten ausgewählt werden. Die Fahrtroutendaten werden von einem Navigationsendgerät empfangen und zur Streckenführung für das Fahrzeug verwendet.

In der EP 1 201 849 A2 wird ein Verfahren und eine Vorrichtung zur Parkraumzuordnung beschrieben. Bei dem Verfahren wird über ein Funkgerät in einem Fahrzeug eine Anfrage nach einer Parkmöglichkeit an die Vorrichtung zur Parkraumzuordnung gestellt. Diese Anfrage enthält Informationen über die aktuelle Position des Fahrzeugs. Die Vorrichtung ermittelt daraus die nächste freie Parkmöglichkeit und übermittelt diese als Zielinformation an das Fahrzeug. Anschließend wird die erwartete Ankunftszeit berechnet und zu einer vorgegebenen Vorlaufzeit vor der erwarteten Ankunftszeit wird überprüft, ob die ermittelte Parkmöglichkeit immer noch zur Verfügung steht. Ist die Parkmöglichkeit frei, besteht die Möglichkeit, diese für das Fahrzeug zu reservieren. Ist die Parkmöglichkeit nicht mehr frei, so ermittelt die Vorrichtung eine neue Parkmöglichkeit und überträgt entsprechende Informationen an das Fahrzeug.

Aus der DE 103 02 504 A1 ist ein Verfahren zum Ermitteln der Reichweite eines Elektrofahrzeugs bekannt. Bei dem Verfahren werden Fahrzeug-, Fahrstrecken- und/oder umweltbezogene Informationen über das Fahrzeug und eine geplante oder eine aktuell zu befahrende Fahrstrecke von einem Fahrzeugcomputer erfasst und verarbeitet. Aus diesen Informationen wird die verbleibende Restreichweite des Elektrofahrzeugs errechnet und angezeigt.

Aus der DE 10 2005 055 243 A1 ist ein Verfahren zur Bestimmung eines energetisch günstigen Streckenverlaufs für ein Fahrzeug bekannt. Bei dem Verfahren wird der Startpunkt und Endpunkt der Strecke eingegeben. Ferner werden fahrzeugbezogene Informationen, insbesondere Beladungsinformationen, bereitgestellt. Die bereitgestellten fahrzeugbezogenen Informationen werden mit gespeicherten Streckendaten in Form fahrzeugbezogener Informationen und Energieverbrauche verglichen. Daraufhin wird eine Fahrtstrecke mit minimiertem Energieverbrauch anhand der gespeicherten Streckendaten in Abhängigkeit von den bereitgestellten fahrzeugbezogenen Informationen sowie dem Start- und Endpunkt ausgewählt. Schließlich werden die Daten zum ausgewählten Streckenverlauf ausgegeben.

Aus der DE 2009 053 982 A1 ist ein System zur Berechnung einer verbrauchsoptimierten Route eines Kraftfahrzeugs bekannt. Das System weist einen Positionsempfänger, einen Verkehrsinformationsempfänger und eine Recheneinheit auf, mithilfe derer eine Route zu einem Zielort berechenbar ist. Die Recheneinheit kann eine verbrauchsoptimierte Route unter Verwendung der fahrzeugindividuellen und/oder fahrerindividuellen verbrauchsrelevanten Daten berechnen.

Aus der EP 1909069 A1 ist ein System zur Reiseroutenplanung für Fahrzeuge bekannt, wobei Termindaten berücksichtigt sind.

Aus der US 5815824 ist ein Navigationssystem für Hybridfahrzeuge bekannt, wobei der Batterieladezustand und die Lage von Energieversorgungseinrichtungen berücksichtigt sind.

Aus der EP 1975562 A2 ist ein Navigationssystem für Elektrofahrzeuge bekannt, wobei die erforderlichen Ladezeiten an Ladestationen verwendet wird, um den Benutzer vorzuschlagen, in der Nähe gelegene Sehenswürdigkeiten besuchen zu gehen.

Der vorliegenden Erfindung liegt das technische Problem zugrunde, ein Verfahren und eine Vorrichtung der eingangs genannten Art bereitzustellen, mit denen in Abhängigkeit von Termindaten eine optimale Reiseroutenplanung durchgeführt werden kann.

Erfindungsgemäß wird dieses Problem durch ein Verfahren und eine Vorrichtung mit den Merkmalen der unabhängigen Ansprüche gelöst. Vorteilhafte Ausgestaltungen und Weiterbildungen ergeben sich aus den abhängigen Ansprüchen.

Das Fahrzeug, für welches die Reiseroutenplanung durchgeführt werden soll, umfasst einen Energiespeicher zum Speichern der Energie zum Antrieb des Fahrzeugs, beispielsweise eine wiederaufladbare Batterie und/oder einen Kraftstofftank. Die Erfindung bezieht sich insbesondere auf die Tagesfahrtenplanung im Bereich der Mobilität mit elektrisch betriebenen Fahrzeugen oder Fahrzeugen, für welche die räumliche Dichte an Energieversorgungseinrichtungen gering ist, wie dies z. B. bei gasbetriebenen Fahrzeugen oder Fahrzeugen mit einem Brennstoffzellenantrieb derzeit der Fall ist. Dabei wird insbesondere auch die Zeit berücksichtigt, die benötigt wird, um den Energiespeicher des Fahrzeugs aufzufüllen.

Gemäß einem ersten Aspekt der Erfindung wird bei dem erfindungsgemäßen Verfahren eine Zielsequenz für die zu planende Reiseroute an eine Recheneinheit übermittelt. Die Recheneinheit ist mit einem Datenspeicher gekoppelt, in dem Daten zu einem Wegenetz und Daten zu geographischen Positionen von Standplätzen, die Parkplätze oder Energieversorgungseinrichtungen umfassen, für das Fahrzeug gespeichert. Die Recheneinheit berechnet eine Routensequenz. Dabei wird eine prognostizierte Restenergiemenge in dem Energiespeicher für das Befahren der Routensequenz berechnet. Ferner werden für die Ziele der Zielsequenz jeweils zu den Zielen zugeordnete Standplätze in der Nähe des jeweiligen Ziels ermittelt. Für jedes Ziel der Zielsequenz wird ein zugeordneter Standplatz bestimmt, wobei bei der Bestimmung die Entfernung des Standplatzes von dem zugeordneten Ziel, die geographische Position des nächsten Ziels oder die geographischen Positionen der Standplätze des nächsten Ziels und/oder die prognostizierte Restenergiemenge in dem Energiespeicher für das Befahren der Routensequenz berücksichtigt wird. Die Routensequenz setzt sich dann aus Routen zwischen Standplätzen aufeinanderfolgender Ziele der Zielsequenz zusammen. Schließlich kann bei dem Verfahren die berechnete Routensequenz ausgegeben oder übertragen werden.

Unter einer Zielsequenz wird im Sinne der Erfindung eine Abfolge von geographischen Positionen verstanden, die nacheinander durch die zu planende Reiseroute verbunden werden. Entsprechend wird unter einer Routensequenz eine Abfolge von Routen verstanden, die Ziele bzw. den Zielen zugeordnete Standplätze miteinander verbindet.

Bei dem erfindungsgemäßen Verfahren gemäß dem ersten Aspekt der Erfindung wird somit bei der Bestimmung der Standplätze, die einem Ziel zugeordnet sind, das Ziel in einen Zielraum erweitert. Dieser Zielraum enthält das Ziel sowie die zugeordneten Standplätze. Die Größe dieses Zielraums kann beispielsweise von dem maximalen Abstand abhängen, den ein Standplatz von dem Ziel entfernt sein darf. Ist der Zielraum bestimmt worden, werden die Standplätze ermittelt, deren geographische Positionen innerhalb des Zielraums liegen. Bei der Auswahl des Standplatzes wird bei dem erfindungsgemäßen Verfahren nicht nur die Entfernung des Standplatzes von dem zugeordneten Ziel berücksichtigt, sondern auch das nächste Ziel sowie ggf. das vorherige Ziel der Routensequenz und die prognostizierte Restenergiemenge in dem Energiespeicher. Bei dem erfindungsgemäßen Verfahren gemäß dem ersten Aspekt wird die Reiseroutenplanung somit vorteilhafterweise unter Berücksichtigung verschiedener möglicher Standplätze für das Fahrzeug beim Erreichen eines Ziels der Zielsequenz durchgeführt. Auf diese Weise lässt sich die Route zwischen den Zielen der Zielsequenz optimieren. Ferner kann ein Auffüllen des Energiespeichers berücksichtigt werden, da ein ausgewählter Standplatz auch eine Energieversorgungseinrichtung umfassen kann. Für die vorgegebene Zielsequenz kann somit eine optimierte Reiseroutenplanung durchgeführt werden.

Gemäß einer Ausgestaltung des erfindungsgemäßen Verfahrens entscheidet die Recheneinheit automatisch in Abhängigkeit von der prognostizierten Restenergiemenge in dem Energiespeicher für das Befahren der Routensequenz, ob als Standplatz ein Parkplatz oder eine Energieversorgungseinrichtung ausgewählt wird. Durch die Auswahl des Standplatzes kann somit vorteilhafterweise sichergestellt werden, dass das Fahrzeug beim Befahren der Routensequenz zu jeder Zeit ausreichend Energiereserven für den Antrieb des Fahrzeugs sowie gegebenenfalls für interne Verbraucher des Fahrzeugs hat. Dies ist insbesondere wichtig, wenn es sich bei dem Fahrzeug um ein Elektrofahrzeug mit einer relativ geringen Reichweite handelt.

Gemäß einer Ausgestaltung des erfindungsgemäßen Verfahrens entscheidet die Recheneinheit ferner automatisch in Abhängigkeit von den Energiekosten zum Auffüllen des Energiespeichers des Fahrzeugs, welche Standplätze ausgewählt werden.

Gemäß einer Weiterbildung des erfindungsgemäßen Verfahrens werden Termindaten, welche geographische Positionen von zumindest einem Teil der Ziele der zu planenden Reiseroute und zugehörige Zeitdaten umfassen, an die Recheneinheit übermittelt. In diesem Fall berücksichtigt die Recheneinheit bei der Bestimmung der Standplätze die prognostizierte Restenergiemenge in dem Energiespeicher für das Befahren der Routensequenz, die Zeitdaten für das Ziel, welches dem Standplatz zugeordnet ist, und die Dauer zum Erhöhen der Energiereserven in dem Energiespeicher mittels der Energieversorgungseinrichtung.

Die Recheneinheit kann somit aus den Termindaten die Ortsinformationen zu der Zielsequenz ermitteln sowie die Zeiten, zu denen das Fahrzeug die Ziele erreichen muss. Ferner kann die Recheneinheit aus den Termindaten die Aufenthaltsdauer des Fahrzeugs bei einem Ziel ermitteln. Die Reiseroute kann insbesondere so geplant werden, dass ein Standplatz gewählt wird, der eine Energieversorgungseinrichtung umfasst, wobei während der Aufenthaltsdauer des Fahrzeugs bei einem Ziel der Energiespeicher aufgefüllt wird. Um die Reiseroute zu optimieren, kann bei dem Verfahren allerdings berücksichtigt werden, dass es nicht unbedingt erforderlich ist, dass der Energiespeicher bei einer Energieversorgungseinrichtung maximal aufgefüllt wird. Wenn das Ende eines Termins erreicht ist, kann ggf. das Auffüllen des Energiespeichers abgebrochen werden, bevor der Energiespeicher maximal aufgefüllt ist. Hierdurch werden vorteilhafterweise unnötige Wartezeiten für den Nutzer vermieden, die durch das Auffüllen des Energiespeichers verursacht werden. Die Standzeit des Fahrzeugs während eines Termins des Nutzers kann optimal zum Auffüllen des Energiespeichers verwendet werden, ohne dass sich zusätzliche Wartezeiten für den Nutzer ergeben.

Gemäß einer Weiterbildung des erfindungsgemäßen Verfahrens ermittelt die Recheneinheit für die Standplätze außerdem den Abstand vom zugeordneten Ziel oder die Dauer, die ein Nutzer für den Weg von dem Standplatz zu dem zugeordneten Ziel benötigt. Die Recheneinheit kann in diesem Fall bei der Bestimmung der Standplätze ferner die ermittelte Dauer bzw. den Abstand sowie die Termindaten berücksichtigen. Dabei kann insbesondere auch berücksichtigt werden, ob der Nutzer zu Fuß von dem Standplatz zum Ziel geht oder auf andere Weise zum Ziel gelangt. Ferner kann eine nutzerabhängige Gehgeschwindigkeit berücksichtigt werden.

Bei dem Verfahren wird mittels der Recheneinheit insbesondere die Routensequenz hinsichtlich des Energieverbrauchs beim Befahren der Routensequenz und/oder hinsichtlich der Zeit zum Befahren der Routensequenz optimiert. Ferner wird sichergestellt, dass die Ziele der Zielsequenz entsprechend den Termindaten erreicht werden, wobei nicht nur die Zeit zum Erreichen des Standplatzes, welches einem Ziel zugeordnet ist, berücksichtigt wird, sondern auch die anschließende Dauer zum Erreichen des Ziels.

Gemäß einer Weiterbildung des erfindungsgemäßen Verfahrens ermittelt die Recheneinheit des Weiteren die Verfügbarkeitswahrscheinlichkeit der Standplätze. Bei der Bestimmung der Standplätze wird dann die Verfügbarkeitswahrscheinlichkeit berücksichtigt. Wenn es relativ unwahrscheinlich ist, dass ein Standplatz zu der benötigten Zeit, die sich aus der Routensequenz ergibt, frei ist, kann ein anderer Standplatz gewählt werden. Dabei kann die Verfügbarkeitswahrscheinlichkeit des Standplatzes in Relation zu der Verschlechterung der Routensequenz durch den anderen Standplatz gesetzt werden. Auf diese Weise kann vorteilhafterweise die Reiseroutenplanung weiter optimiert werden. Die Verfügbarkeitswahrscheinlichkeit der Standplätze wird insbesondere anhand der Ankunftszeit des Fahrzeugs am Standplatz und der Aufenthaltsdauer am Standplatz ermittelt. Ferner kann die Recheneinheit beispielsweise auf historische Daten zurückgreifen, die statistisch angeben, zu welchen Zeiten die einzelnen Standplätze in der Vergangenheit belegt waren bzw. frei waren. Außerdem können bereits erfolgte Buchungen von Dritten für die Standplätze bei der Verfügbarkeitswahrscheinlichkeit berücksichtigt werden.

Des Weiteren kann bei der Berechnung der Routensequenz das Fahrverhalten eines bestimmten Nutzers prognostiziert werden. Beispielsweise kann für einen bestimmten Fahrer aus historischen Daten ermittelt werden, wie schnell bzw. langsam er fährt. Daraus können Profile für bestimmte Fahrer abgeleitet werden.

Gemäß einer Ausgestaltung des erfindungsgemäßen Verfahrens berechnet die Recheneinheit die prognostizierte Restenergiemenge in dem Energiespeicher anhand des prognostizierten Energieverbrauchs beim Befahren der Routensequenz. Dabei wird zum einen der prognostizierte Energieverbrauch zum Antrieb des Fahrzeugs und zum anderen der prognostizierte Energieverbrauch interner Verbraucher des Fahrzeugs berücksichtigt. Dieser prognostizierte Energieverbrauch wird dann bei der prognostizierten Restenergiemenge im Energiespeicher während des Befahrens der Routensequenz berücksichtigt. Auf diese Weise wird vorteilhafterweise bei einem Elektrofahrzeug berücksichtigt, dass der Energieverbrauch auch vom Ladezustand der Batterie abhängt. Ist der Ladezustand einer Batterie nämlich niedriger, ergibt sich beim Befahren einer Route nämlich eine stärkere Änderung des Ladezustands als bei einem höheren Ladezustand der Batterie. Wird das Fahrzeug mittels eines Kraftstoffs angetrieben, wird in diesem Fall berücksichtigt, dass der Kraftstoffverbrauch aufgrund der Änderung des Gewichts des Fahrzeugs auch von der Restkraftstoffmenge im Fahrzeug abhängt.

Bei der Prognose des Energieverbrauchs der internen Verbraucher des Fahrzeugs können Wettervorhersagen oder die Jahreszeit berücksichtigt werden, zu welcher die Routensequenz abgefahren werden soll. Wenn sich ergibt, dass die Umgebungstemperatur während des Befahrens der Routensequenz voraussichtlich sehr hoch ist, kann bei der Prognose des Energieverbrauchs aufgrund interner Verbraucher berücksichtigt werden, dass mit hoher Wahrscheinlichkeit die Klimaanlage des Fahrzeugs während des Befahrens der Routensequenz eingeschaltet ist. Außerdem können Merkmale des Wegenetzes der Routensequenz, insbesondere Fahrbahnsteigungen und Verzögerungen bzw. Beschleunigungen auf Grund von Kurven, berücksichtigt werden. Diese Merkmale des Wegenetzes haben Einfluss auf den Energieverbrauch beim Befahren der Routensequenz.

Gemäß einer Weiterbildung des erfindungsgemäßen Verfahrens werden der Recheneinheit nutzerspezifische Nebenbedingungen übertragen. Die Recheneinheit berücksichtigt diese nutzerspezifischen Nebenbedingungen bei der Ermittlung der Standplätze. Als Nebenbedingungen kann der Nutzer Präferenzen angeben. Beispielsweise kann der Nutzer angeben, mit welcher Priorität sichergestellt werden soll, dass eine bestimmte Restenergiemenge im Energiespeicher immer gespeichert ist. Diese Nebenbedingung kann dem Nutzer eine Sicherheit vermitteln, bei etwaigen unvorhergesehenen Änderungen der Termine ausreichend Energie im Energiespeicher des Fahrzeugs zu haben, um auf diese Änderungen reagieren zu können. Ferner kann der Nutzer die Priorität dafür angeben, dass Termine pünktlich eingehalten werden. Alternativ kann er eine bestimmte akzeptable Verspätungsdauer angeben. In diesem Fall kann bei der Reiseroutenplanung auch berücksichtigt werden, dass die Termine bei der Routensequenz zwar nicht exakt eingehalten werden können, die optimale Routensequenz jedoch sehr viel besser als die nächstbeste Routensequenz ist, so dass geringfügige Verspätungen im Rahmen der Vorgaben des Nutzers toleriert werden.

Des Weiteren kann der Nutzer Prioritäten hinsichtlich der Standplätze angeben. Beispielsweise kann er als Nebenbedingung einstellen, wie groß die maximale Entfernung eines Standplatzes von dem Ziel ist. Ferner kann er eine Präferenz für einen bestimmten Parkplatztyp, wie zum Beispiel einen Behindertenparkplatz, angeben.

Nachdem durch das erfindungsgemäße Verfahren eine Routensequenz berechnet wurde, wird die berechnete Routensequenz ausgegeben, beispielsweise mittels einer Anzeige in Verbindung mit einer geographischen Karte. Ferner können die zu der Routensequenz gehörigen Standplätze automatisch reserviert werden, indem entsprechende Daten an eine entsprechende Einrichtung zum Reservieren von Standplätzen übertragen wird. Des Weiteren kann die berechnete Routensequenz an eine Einrichtung des Fahrzeugs übertragen werden.

Gemäß einer Weiterbildung des erfindungsgemäßen Verfahrens wird während des Befahrens der berechneten Routensequenz die Restenergiemenge im Energiespeicher des Fahrzeugs erfasst und mit der prognostizierten Restenergiemenge des Fahrzeugs für eine entsprechende Routenposition verglichen. Falls die Abweichung der erfassten Restenergiemenge von der prognostizierten Restenergiemenge einen Grenzwert überschreitet, wird die Routensequenz auf Basis der erfassten Restenergiemenge neu berechnet. Das erfindungsgemäße Verfahren dient somit nicht nur der Durchführung einer Reiseroutenplanung vor Antritt der Fahrt. Vielmehr kann auch während der Fahrt überprüft werden, ob die bei der Berechnung der Routensequenz gemachten Prognosen zutreffend waren. Insbesondere die Prognosen zum Energieverbrauch des Fahrzeugs beim Befahren der Route, d.h. die prognostizierte Restenergiemenge bei den einzelnen Routenpositionen, werden mit der tatsächlichen Restenergiemenge verglichen. Bei Abweichungen, die einen bestimmten Grenzwert, der auch 0 sein kann, überschreiten, wird die Routensequenz neu berechnet. Diese Berechnung kann entsprechend der vorab durchgeführten Berechnung durchgeführt werden. Der Grenzwert kann z.B. bei einer Abweichung von 5% oder 10% liegen.

Des Weiteren kann überprüft werden, ob aufgrund der Abweichung der tatsächlichen Restenergiemenge von der prognostizierten Restenergiemenge ein bestimmtes Ziel der Zielsequenz nicht mehr erreicht werden kann, da die Energie zum Antrieb des Fahrzeugs hierfür nicht mehr ausreicht. In einem solchen Fall kann die Routensequenz insbesondere so geändert werden, dass als Zwischenziel die geographische Position einer Energieversorgungseinrichtung in die Routensequenz eingefügt wird. Hierdurch wird vorteilhafterweise sichergestellt, dass die Ziele der Zielsequenz in jedem Fall erreicht werden können, selbst wenn sie zu einem späteren Zeitpunkt erreicht werden, so dass eventuell bestimmte Termine zeitlich nicht eingehalten werden können.

Des Weiteren können auch hinsichtlich anderer Annahmen, die bei der Vorausberechnung der Routensequenz getroffen wurden, Abweichungen von den tatsächlichen Werten beim Befahren der Routensequenz auftreten. Beispielsweise kann überprüft werden, ob bestimmte Positionen der Routensequenz in Übereinstimmung mit der Prognose zeitlich rechtzeitig erreicht werden. Dabei können auch Daten berücksichtigt werden, die aus einem anderen Fahrzeug heraus generiert worden sind, welches aktuell am Verkehrsgeschehen teilnimmt (sogenannte XFCD - extended floating car data). Des Weiteren können auch Daten einer Fahrzeug-zu-Fahrzeug oder Fahrzeug-zu-X Kommunikation berücksichtigt werden. Auch in diesem Fall kann die Routensequenz bei etwaigen Abweichungen neu berechnet werden. Außerdem können aktuelle Verkehrsdaten berücksichtigt werden. Wenn diese Verkehrsdaten von den Annahmen bei der Vorausberechnung der Routensequenz abweichen, und gegebenenfalls bestimmte Ziele zeitlich oder energetisch nicht bzw. nicht rechtzeitig erreicht werden können, kann eine angepasste Routensequenz berechnet werden.

Des Weiteren können die Verfügbarkeitswahrscheinlichkeiten für Standplätze auch noch während der Fahrt durch eine Datenübertragung per Funk aktualisiert werden. Die Recheneinheit kann insbesondere aktuelle Daten zur Belegung der Standplätze, einschließlich der voraussichtlichen Dauer der Belegung, berücksichtigen und gegebenenfalls die Routensequenz so anpassen, dass andere Standplätze ausgewählt werden als die bei der im Voraus berechneten Routensequenz.

Schließlich kann die Route auch kontinuierlich und nicht in Abhängigkeit von bestimmten Ereignissen während der Fahrt umgeplant werden.

Gemäß dem ersten Aspekt der Erfindung wird ferner eine Vorrichtung zur Reiseroutenplanung für ein Fahrzeug bereitgestellt. Das Fahrzeug umfasst einen Energiespeicher zum Speichern der Energie zum Antrieb des Fahrzeugs. Die Vorrichtung umfasst eine Recheneinheit und einen mit der Recheneinheit gekoppelten Datenspeicher, in dem Daten zu einem Wegenetz und Daten zu geographischen Positionen von Standplätzen, die Parkplätze oder Energieversorgungseinrichtungen umfassen, für das Fahrzeug gespeichert sind. Des Weiteren umfasst die Vorrichtung eine mit der Recheneinheit gekoppelte Schnittstelle, über welche eine Zielsequenz für die zu planende Reiseroute an die Recheneinheit übermittelbar ist. Optional kann ferner eine mit der Recheneinheit gekoppelte Ausgabeeinheit vorgesehen sein, mittels welcher eine von der Recheneinheit berechnete Routensequenz ausgebbar, insbesondere anzeigbar, ist. Alternativ oder zusätzlich kann auch eine Schnittstelle zur Übertragung der Daten zu der berechneten Routensequenz vorgesehen sein. Bei der erfindungsgemäßen Vorrichtung ist mittels der Recheneinheit eine Routensequenz berechenbar, wobei die prognostizierte Restenergiemenge in dem Energiespeicher für das Befahren der Routensequenz berechnet wird. Für die Ziele der Zielsequenz werden jeweils zu den Zielen zugeordnete Standplätze in der Nähe des jeweiligen Ziels ermittelt. Für jedes Ziel der Zielsequenz wird ein zugeordneter Standplatz bestimmt, wobei bei der Bestimmung die Entfernung des Standplatzes von dem zugeordneten Ziel, die geographische Position des nächsten Ziels oder die geographischen Positionen der Standplätze des nächsten Ziels und/oder die prognostizierte Restenergiemenge in dem Energiespeicher für das Befahren der Routensequenz berücksichtigt werden. Die Routensequenz setzt sich dann aus Routen zwischen Standplätzen aufeinanderfolgender Ziele der Zielsequenz zusammen.

Die erfindungsgemäße Vorrichtung ist insbesondere geeignet, das vorstehend beschriebene erfindungsgemäße Verfahren auszuführen. Sie weist daher auch dieselben Vorteile wie das erfindungsgemäße Verfahren auf.

Gemäß einer Weiterbildung der erfindungsgemäßen Vorrichtung umfasst sie ein fahrzeugexternes Modul und ein fahrzeuginternes Modul, wobei das fahrzeugexterne Modul die Recheneinheit umfasst. Das fahrzeugexterne und das fahrzeuginterne Modul sind über eine Schnittstelle zumindest zeitweise datentechnisch miteinander gekoppelt, so dass zumindest die Routensequenz von dem fahrzeugexternen Modul an das fahrzeuginterne Modul übertragbar ist. Das fahrzeuginterne Modul umfasst eine weitere Recheneinheit, eine weitere Ausgabeeinheit, einen Speicher zum Speichern einer von dem fahrzeugexternen Modul übertragenen Routensequenz und einen Sensor zum Erfassen der Restenergiemenge im Energiespeicher des Fahrzeugs. Mittels der weiteren Recheneinheit ist während des Befahrens der berechneten Routensequenz die erfasste Restenergiemenge im Energiespeicher des Fahrzeugs mit der prognostizierten Restenergiemenge des Fahrzeugs für eine entsprechende Routenposition der in dem Speicher gespeicherten Routensequenz vergleichbar. Falls die Abweichung der erfassten Restenergiemenge von der prognostizierten Restenergiemenge einen Grenzwert überschreitet, ist die Routensequenz auf Basis der erfassten Restenergiemenge neu berechenbar und über die Ausgabeeinheit ausgebbar. Vorteilhafterweise ist es somit möglich, während der Fahrt die im Voraus berechnete Routensequenz anzupassen, wenn die tatsächliche Restenergiemenge im Energiespeicher des Fahrzeugs von der prognostizierten Restenergiemenge des Fahrzeugs abweicht. Ferner können auch bei Abweichungen anderer Parameter, die bei der Vorausberechnung der Routensequenz verwendet worden sind, Anpassungen der Routensequenz von der weiteren Recheneinheit im Fahrzeug vorgenommen werden, wie es vorstehend mit Bezug zu dem erfindungsgemäßen Verfahren erläutert wurde.

Die Reiseroutenplanung, d. h. insbesondere die Berechnung, Planung und Optimierung der Reiseroute, kann durch eine fahrzeugexterne, eine fahrzeuginterne oder aufgeteilt auf eine fahrzeugexterne und eine fahrzeuginterne Einrichtung erfolgen.

Gemäß einem zweiten Aspekt der Erfindung wird ein Verfahren zur Durchführung einer Reiseroutenplanung für ein Fahrzeug bereitgestellt, bei dem Termindaten, welche geographische Positionen von Zielen der zu planenden Reiseroute und zugehörige Zeitdaten umfassen, an eine Recheneinheit übermittelt werden, die mit einem Datenspeicher gekoppelt ist, in dem Daten zu einem Wegenetz für das Fahrzeug und Daten zu den geographischen Positionen von Energieversorgungseinrichtungen gespeichert sind. Die Recheneinheit prüft bei dem Verfahren, ob eine Routensequenz berechenbar ist, welche die geographischen Positionen der zu den Termindaten gehörigen Zielen so verbindet, dass die Ziele zu den zugehörigen Zeitdaten der Termindaten erreicht werden, wobei die prognostizierte Restenergiemenge in dem Energiespeicher des Fahrzeugs für das Befahren der Routensequenz bestimmt und berücksichtigt wird. Falls mittels der Recheneinheit keine solche Routensequenz berechenbar ist, bestimmt die Recheneinheit angepasste Termindaten, für welche eine solche Routensequenz berechenbar ist. Die angepassten Termindaten werden dann ausgegeben.

Bei dem erfindungsgemäßen Verfahren gemäß dem zweiten Aspekt wird somit nicht nur berücksichtigt, ob die Energiereserven des Fahrzeugs ausreichen, um zu den Zielen der Zielsequenz zu gelangen. Es wird auch überprüft, ob in zeitlicher Hinsicht die Termine mit einer berechneten Routensequenz eingehalten werden können. Ist dies nicht der Fall, wird bei dem erfindungsgemäßen Verfahren gemäß dem zweiten Aspekt eine Änderung der Termindaten bestimmt, zu der es eine Routensequenz gibt, die in zeitlicher Hinsicht keinen Konflikt zu den Termindaten verursacht. Gleichzeitig wird jedoch auch die prognostizierte Restenergiemenge im Energiespeicher des Fahrzeugs berücksichtigt, damit sichergestellt wird, dass die Ziele in energetischer Hinsicht erreicht werden können. Bei der Prognose der Restenergiemenge wird auch berücksichtigt, dass die in dem Energiespeicher des Fahrzeugs gespeicherte Energiemenge bei den geographischen Positionen der Energieversorgungseinrichtungen erhöht werden kann, wobei auch in diesem Fall nicht bei jeder Energieversorgungseinrichtung der Energiespeicher des Fahrzeugs vollständig aufgefüllt werden muss.

Bei der Prüfung, ob eine Routensequenz berechenbar ist, welche keinen Konflikt zu den Termindaten verursacht, kann die Recheneinheit insbesondere verschiedene mögliche Alternativen ausprobieren und diese darauf überprüfen, ob die geographischen, die zeitlichen und die energetischen Nebenbedingungen erfüllt sind.

Bei der Prüfung, ob die Ziele zu den entsprechenden Anfangszeiten der Termine erreicht werden können, wird eine Dauer zum Erreichen des Ziels beim Befahren der Routensequenz berechnet. In diese Berechnung der Routendauer können vielfältige Faktoren einfließen. Die Routendauer kann beispielsweise von der Tageszeit, dem Wochentag, etwaigen Feiertagen und/oder dem voraussichtlichen Verkehrsaufkommen abhängen. Ferner kann auf gespeicherte historische Daten zurückgegriffen werden, um die Routendauer für das Befahren während einer bestimmten Tageszeit genauer berechnen zu können.

Gemäß einer Weiterbildung des erfindungsgemäßen Verfahrens werden bei den angepassten Termindaten geographischen Positionen von Zielen neue Zeitdaten zugeordnet. Jedes Ziel der Termindaten wird somit weiterhin von der Routensequenz angefahren, jedoch zu anderen Zeiten. Hierdurch kann vorteilhafterweise eine Reiseroutenplanung bereitgestellt werden, bei welcher der Nutzer die beabsichtigten Termine wahrnehmen kann, sie allerdings verschieben muss. Auf welche Zeiten die Termine verschoben werden sollen, kann der Nutzer einfach durch die Reiseroutenplanung erfahren. Nachdem die Termine verschoben worden sind, liegt eine Routensequenz vor, die sicherstellt, dass die Ziele in zeitlicher und energetischer Hinsicht erreicht werden können.

Falls es nicht möglich ist, eine Routensequenz zu finden, welche die Ziele auch mit geänderten Zeitdaten miteinander durch eine Routensequenz verbindet, kann bei dem erfindungsgemäßen Verfahren auch vorgeschlagen werden, dass ein Ziel oder mehrere Ziele gestrichen oder verschoben werden oder die Reihenfolge der Ziele geändert wird. Auch hierdurch wird dem Nutzer die Anpassung seiner Termine erleichtert, da berechnet wird, welches Ziel beispielsweise gestrichen oder verschoben werden muss, um eine Routensequenz zu erhalten, welche die verbleibenden Ziele ohne zeitlichen oder energetischen Konflikt miteinander verbindet. Diese Änderung der Zeitdaten kann auch interaktiv mit dem Nutzer erfolgen.

Bei dem erfindungsgemäßen Verfahren optimiert die Recheneinheit insbesondere bei der Berechnung der Routensequenz die für den Nutzer des Fahrzeugs nutzbare Zeit. Diese nutzbare Zeit kann beispielsweise dadurch erhöht werden, dass die Energiemenge in dem Energiespeicher des Fahrzeugs während einer Standzeit des Fahrzeugs während eines Termins des Nutzers erhöht wird. Falls es aus energetischer Sicht erforderlich ist, wählt die Recheneinheit für die Routensequenz die Position einer Energieversorgungseinrichtung in der Nähe eines Ziels, insbesondere bei einem Standplatz des Ziels, aus, damit der Energiespeicher des Fahrzeugs während der Standzeit des Fahrzeugs während eines Termins aufgeladen werden kann.

Gemäß dem zweiten Aspekt der Erfindung wird ferner eine Vorrichtung zur Reiseroutenplanung für ein Fahrzeug, das einen Energiespeicher zum Speichern der Energie zum Antrieb des Fahrzeugs umfasst, vorgeschlagen, welche eine Recheneinheit und einen mit der Recheneinheit gekoppelten Datenspeicher aufweist, in dem Daten zu einem Wegenetz und Daten zu geographischen Positionen von Energieversorgungseinrichtungen für das Fahrzeug gespeichert sind. Des Weiteren ist eine mit der Recheneinheit gekoppelte Schnittstelle vorgesehen, über welche Termindaten, welche geographische Positionen von Zielen der zu planenden Reisroute und zugehörige Zeitdaten umfassen, an die Recheneinheit übermittelbar sind. Ferner umfasst die Vorrichtung eine mit der Recheneinheit gekoppelte Ausgabeeinheit, mittels welcher eine von der Recheneinheit berechnete Routensequenz und/oder angepasste Termindaten ausgebbar sind. Die erfindungsgemäße Vorrichtung gemäß dem zweiten Aspekt der Erfindung ist dadurch gekennzeichnet, dass mittels der Recheneinheit prüfbar ist, ob eine Routensequenz berechenbar ist, welche die geographischen Positionen der zu den Termindaten gehörigen Ziele so verbindet, dass die Ziele zu den zugehörigen Zeitdaten der Termindaten erreicht werden, wobei die prognostizierte Restenergiemenge in dem Energiespeicher des Fahrzeugs für das Befahren der Routensequenz bestimmt und berücksichtigt wird. Falls keine solche Routensequenz berechenbar ist, sind mit der Recheneinheit angepasste Termindaten bestimmbar, für welche eine solche Routensequenz berechenbar ist.

Die erfindungsgemäße Vorrichtung gemäß dem zweiten Aspekt der Erfindung ist insbesondere zum Ausführen des erfindungsgemäßen Verfahrens gemäß dem zweiten Aspekt der Erfindung geeignet. Sie weist somit auch dieselben Vorteile wie das Verfahren gemäß dem zweiten Aspekt der Erfindung auf.

Gemäß einer Weiterbildung der erfindungsgemäßen Vorrichtung umfasst diese ein fahrzeugexternes Modul und ein fahrzeuginternes Modul, wobei das fahrzeugexterne Modul die Recheneinheit umfasst. Das fahrzeugexterne und das fahrzeuginterne Modul sind über eine Schnittstelle zumindest zeitweise datentechnisch miteinander gekoppelt, so dass zumindest eine Routensequenz von dem fahrzeugexternen Modul an das fahrzeuginterne Modul übertragbar ist. Das fahrzeuginterne Modul umfasst eine weitere Recheneinheit, eine weitere Ausgabeeinheit, einen Speicher zum Speichern einer vom fahrzeugexternen Modul übertragenen Routensequenz und einen Sensor zum Erfassen der Restenergiemenge im Energiespeicher des Fahrzeugs. Mittels der weiteren Recheneinheit ist während des Befahrens der berechneten Routensequenz die erfasste Restenergiemenge im Energiespeicher des Fahrzeugs mit der prognostizierten Restenergiemenge des Fahrzeugs für eine entsprechende Routenposition der in dem Speicher gespeicherten Routensequenz vergleichbar. Falls die Abweichung der erfassten Restenergiemenge von der prognostizierten Restenergiemenge einen Grenzwert überschreitet, ist mittels der weiteren Recheneinheit prüfbar, ob die Ziele der Routensequenz zu den zugehörigen Zeitdaten der Termindaten weiterhin erreicht werden, wobei die prognostizierte Restenergiemenge in dem Energiespeicher des Fahrzeugs für das Befahren der Routensequenz bestimmt wird. Falls die Prüfung ergibt, dass die Ziele der Routensequenz nicht zu den zugehörigen Zeitdaten erreicht werden können, berechnet die weitere Recheneinheit zur Ausgabe über die weitere Ausgabeeinheit eine angepasste Routensequenz oder bestimmte angepasste Termindaten.

Die vorgenannten Merkmale des erfindungsgemäßen Verfahrens und der erfindungsgemäßen Vorrichtung gemäß dem ersten Aspekt der Erfindung können einzeln oder zusammen mit dem Verfahren und der Vorrichtung gemäß dem zweiten Aspekt der Erfindung kombiniert werden. Umgekehrt können auch die einzelnen Merkmale des Verfahrens und der Vorrichtung des zweiten Aspektes der Erfindung einzeln oder zusammen mit dem Verfahren und der Vorrichtung gemäß dem ersten Aspekt der Erfindung kombiniert werden.

Gemäß einem dritten Aspekt der Erfindung wird ein Verfahren zum Durchführen einer Reiseroutenplanung für ein Fahrzeug bereitgestellt, bei dem eine Zielsequenz für die zu planende Reiseroute an eine Recheneinheit übermittelt wird, die mit einem Datenspeicher gekoppelt ist, in dem Daten zu einem Wegenetz für das Fahrzeug gespeichert sind. Die Recheneinheit berechnet anschließend eine Routensequenz, welche die Ziele der Zielsequenz verbindet. Ferner wird eine prognostizierte Restenergiemenge in dem Energiespeicher für das Befahren der Routensequenz berechnet. Des Weiteren werden zumindest für die geographische Position des Fahrzeugs bei einem bestimmten Ziel der Ziele der Zielsequenz auf Basis der prognostizierten Restenergiemenge des Energiespeichers des Fahrzeugs und auf Basis des gespeicherten Wegenetzes und eines prognostizierten Energieverbrauchs beim Befahren des Wegenetzes die Punkte des Wegenetzes ermittelt, die von dem einen bestimmten Ziel aus mit der prognostizierten Restenergiemenge bei diesem bestimmten Ziel noch erreichbar sind. Für zumindest das bestimmte Ziel der Zielsequenz wird eine graphische Kartendarstellung erzeugt, bei welcher die geographische Position dieses Zieles und die Punkte des Wegenetzes visualisiert werden, die von diesem Ziel aus mit der prognostizierten Restenergiemenge des Fahrzeugs bei diesem Ziel noch erreichbar sind.

Durch das erfindungsgemäße Verfahren gemäß dem dritten Aspekt wird somit eine Visualisierung der Restreichweite zu einer vorab berechneten Routensequenz erzeugt. Es wird insbesondere für jedes Ziel der Zielsequenz auf einer geographischen Karte ein Bereich gekennzeichnet, welcher von diesem Ziel aus mit der Restenergiemenge im Energiespeicher des Fahrzeugs erreicht werden kann. Durch diese Visualisierung wird dem Nutzer schon während der Planung der Routensequenz, d.h. vor Fahrtantritt, für jedes Ziel ein Eindruck dafür vermittelt, welche Bereiche er mit den jeweiligen Energiereserven des Fahrzeugs noch erreichen kann. Diese Darstellung beugt insbesondere einer Unsicherheit eines Nutzers bei der Verwendung von Elektrofahrzeugen vor. Außerdem kann der Nutzer anhand dieser Visualisierung einfach und intuitiv noch Einfluss auf die Routenplanung nehmen.

Die Visualisierung erfolgt beispielsweise dadurch, dass eine Fläche auf der graphischen Kartendarstellung abgegrenzt wird, wobei die abgegrenzte Fläche die erreichbaren Punkte des Wegenetzes enthält. Ferner kann die Visualisierung durch eine geschlossene Kontur erfolgen. Gemäß einer Weiterbildung des erfindungsgemäßen Verfahrens sind in dem Datenspeicher ferner Daten zur geographischen Positionen von Energieversorgungseinrichtungen für das Fahrzeug gespeichert. Bei der graphischen Kartendarstellung werden die geographischen Positionen von Energieversorgungseinrichtungen visualisiert, die von dem bestimmten Ziel aus mit der prognostizierten Restenergiemenge des Fahrzeugs noch erreichbar sind. Vorteilhafterweise kann der Nutzer auf diese Weise einfach und intuitiv erkennen, ob Energieversorgungseinrichtungen beim Befahren der Routensequenz von einem bestimmten Ziel aus erreicht werden können, so dass der Energiespeicher des Fahrzeugs jederzeit wieder aufgefüllt werden kann.

Gemäß einer Weiterbildung des erfindungsgemäßen Verfahrens enthält die Routensequenz als Zwischenziel bzw. als Standplatz die geographische Position einer Energieversorgungseinrichtung. Für dieses Zwischenziel wird eine graphische Kartendarstellung erzeugt, bei welcher die geographische Position dieses Zwischenziels und die Punkte des Wegenetzes visualisiert werden, die vor dem Auffüllen des Energiespeichers bei der Energieversorgungseinrichtung noch erreichbar sind, und ferner die Punkte des Wegenetzes visualisiert werden, die nach dem Auffüllen des Energiespeichers bei der Energieversorgungseinrichtung erreichbar sind. Auf diese Weise kann dem Nutzer vermittelt werden, wie die Restreichweite vor dem Auffüllen des Energiespeichers und nach dem Auffüllen des Energiespeichers ist.

Gemäß einer Ausgestaltung des erfindungsgemäßen Verfahrens wird eine Referenzposition erfasst. Bei der Berechnung der Routensequenz wird sichergestellt, dass die Referenzposition immer innerhalb der Restreichweite des Fahrzeugs liegt. Die Referenzposition kann beispielsweise der Wohnsitz oder die Arbeitsstätte des Nutzers sein. Bei der Berechnung der Routensequenz wird somit vorteilhafterweise sichergestellt, dass der Nutzer jederzeit während des Befahrens der Routensequenz zu dieser Referenzposition zurückgelangen kann. Dabei liegt die Referenzposition auch dann innerhalb der Restreichweite des Fahrzeugs, wenn das Fahrzeug zum Erreichen der Referenzposition als Zwischenziel eine Energieversorgungseinrichtung für das Fahrzeug anfahren muss. Auch in diesem Fall ist nämlich sichergestellt, dass die Referenzposition aus energetischer Sicht erreichbar ist.

Gemäß einer Weiterbildung des erfindungsgemäßen Verfahrens wird während des Befahrens der berechneten Routensequenz die Restenergiemenge im Energiespeicher des Fahrzeugs erfasst und zumindest beim Erreichen eines Ziels der Zielsequenz eine angepasste graphische Kartendarstellung erzeugt, bei welcher die geographische Position dieses Ziels und die Punkte des Wegenetzes visualisiert werden, die von diesem Ziel aus mit der erfassten Restenergiemenge im Energiespeicher des Fahrzeugs erreicht werden können. Auf diese Weise wird die Restenergiemenge, welche bei der Vorabberechnung der Routensequenz zugrunde gelegt wurde, anhand der tatsächlich erfassten Restenergiemenge im Fahrzeug überprüft. Bei etwaigen Abweichungen wird eine angepasste Kartendarstellung mit der Visualisierung der Restreichweite dem Nutzer angezeigt, so dass dieser einfach und intuitiv erkennen kann, ob die weiteren Ziele der Zielsequenz noch innerhalb der Restreichweite liegen. Ist dies nicht der Fall, kann der Nutzer oder die Recheneinheit automatische Anpassungen der Routensequenz vornehmen, wie dies bereits mit Bezug zu den anderen Aspekten der Erfindung erläutert wurde.

Gemäß dem dritten Aspekt der Erfindung wird ferner eine Vorrichtung zur Reiseroutenplanung für ein Fahrzeug, das einen Energiespeicher zum Speichern der Energie zum Antrieb des Fahrzeugs umfasst, bereitgestellt. Die Vorrichtung weist eine Recheineinheit und einen mit der Recheneinheit gekoppelten Datenspeicher auf, in dem Daten zu einem Wegenetz für das Fahrzeug gespeichert sind. Ferner weist die Vorrichtung eine mit der Recheneinheit gekoppelten Schnittstelle auf, über welche eine Zielsequenz für die zu planende Reiseroute an die Recheneinheit übermittelbar ist. Ferner ist mit der Recheneinheit eine Ausgabeeinheit gekoppelt. Bei der erfindungsgemäßen Vorrichtung gemäß dem dritten Aspekt der Erfindung ist mittels der Recheneinheit eine prognostizierte Restenergiemenge in dem Energiespeicher für das Befahren der Routensequenz berechenbar. Zumindest für die geographische Position des Fahrzeugs bei einem bestimmten Ziel der Ziele der Zielsequenz sind auf Basis der prognostizierten Restenergiemenge des Energiespeichers des Fahrzeugs und auf Basis des gespeicherten Wegenetzes und eines prognostizierten Energieverbrauchs beim Befahren des Wegenetzes die Punkte des Wegenetzes ermittelbar, die von dem einen bestimmten Ziel aus mit der prognostizierten Restenergiemenge bei diesem bestimmten Ziel noch erreichbar sind. Für zumindest das bestimmte Ziel der Zielsequenz ist eine graphische Kartendarstellung erzeugbar und mittels der Ausgabeeinheit ausgebbar, bei welcher die geographische Position dieses Ziels und die Punkte des Wegenetzes visualisiert werden, die von diesem Ziel aus mit der prognostizierten Restenergiemenge des Fahrzeugs bei diesem Ziel noch erreichbar sind.

Diese Vorrichtung gemäß dem dritten Aspekt der Erfindung ist insbesondere ausgebildet, das Verfahren gemäß dem dritten Aspekt der Erfindung auszuführen. Die Vorrichtung weist somit auch dieselben Vorteile wie das Verfahren gemäß dem dritten Aspekt der Erfindung auf.

Gemäß einer Weiterbildung der erfindungsgemäßen Vorrichtung weist diese ein fahrzeugexternes Modul und ein fahrzeuginternes Modul auf, wobei das fahrzeugexterne Modul die Recheneinheit umfasst. Das fahrzeugexterne und das fahrzeuginterne Modul sind über eine Schnittstelle zumindest zeitweise datentechnisch miteinander gekoppelt, so dass zumindest die Routensequenz von dem fahrzeugexternen Modul an das fahrzeuginterne Modul übertragbar ist. Das fahrzeuginterne Modul umfasst eine weitere Recheneinheit, eine weitere Ausgabeeinheit, einen Speicher zum Speichern einer von dem fahrzeuginternen an das fahrzeugexterne Modul übertragenen Routensequenz und einen Sensor zum Erfassen der Restenergiemenge im Energiespeicher des Fahrzeugs. Mittels der weiteren Rechnereinheit ist während des Befahrens der berechneten Routensequenz die erfasste Restenergiemenge im Energiespeicher des Fahrzeugs mit der prognostizierten Restenergiemenge des Fahrzeugs für eine entsprechende Routensequenz der in dem Speicher gespeicherten Routensequenz vergleichbar. Falls die Abweichung der erfassten Restenergiemenge von der prognostizierten Restenergiemenge einen Grenzwert überschreitet, wird eine angepasste graphische Kartendarstellung erzeugt, bei welcher die geographische Position eines Ziels und die Punkte des Wegenetzes visualisiert werden, die von diesem Ziel aus mit der erfassten Restenergiemenge im Energiespeicher des Fahrzeugs erreicht werden können. Auf diese Weise erreicht man vorteilhafterweise eine Anpassung der Visualisierung der Restreichweite des Fahrzeugs beim Erreichen eines Ziels in Abhängigkeit von der tatsächlichen Restenergiemenge im Fahrzeug.

Die Merkmale des Verfahrens und der Vorrichtung gemäß dem dritten Aspekt der Erfindung können einzeln oder in Kombination auch in Verbindung mit den Verfahren und den Vorrichtungen der ersten beiden Aspekte der Erfindung verwendet werden. Umgekehrt können auch die Merkmale der Verfahren der Vorrichtungen der beiden ersten Aspekte der Erfindung einzeln oder in Kombination mit den Verfahren und der Vorrichtung gemäß dem dritten Aspekt der Erfindung verwendet werden.

Die Erfindung wird nun anhand eines Ausführungsbeispiels mit Bezug zu den Zeichnungen erläutert.
- Figur 1: zeigt schematisch ein Ausführungsbeispiel der erfindungsgemäßen Vorrichtung zum Durchführen einer Reiseroutenplanung für ein Fahrzeug,
- Figur 2: zeigt eine Darstellung zur Veranschaulichung der Ziele sowie der den Zielen zugeordneten Standplätze,
- Figur 3: zeigt ein Ausführungsbeispiel des erfindungsgemäßen Verfahrens zum Durchführen einer Reiseroutenplanung für ein Fahrzeug,
- Figur 4: zeigt eine graphische Darstellung mit einer Kurve, welche die Restenergiemenge beim Befahren der Routensequenz zeigt, und
- die Figuren 5 bis 12: zeigen graphische Kartendarstellungen mit Zusatzinformationen zu der Routensequenz.

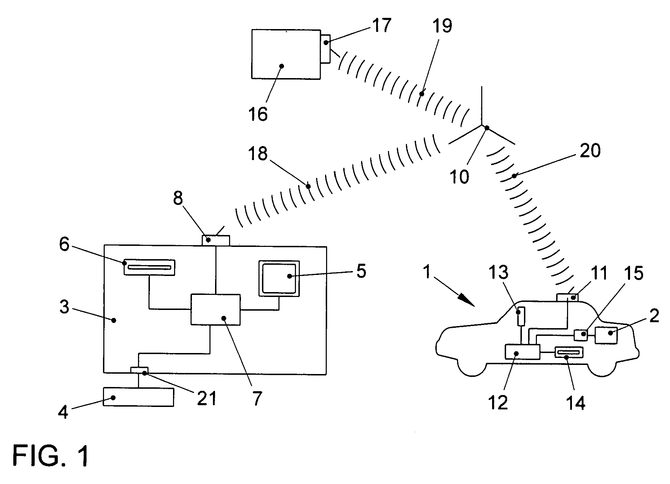

In Figur 1 ist schematisch der grundsätzliche Aufbau eines Ausführungsbeispiels der erfindungsgemäßen Vorrichtung gezeigt. Die Vorrichtung umfasst ein fahrzeuginternes Modul und ein fahrzeugexternes Modul, die zumindest zeitweise Daten miteinander austauschen können.

Das fahrzeugexterne Modul kann einen Computer 3 umfassen, welcher über eine Schnittstelle 21 mit einer Eingabeeinheit 4 verbunden ist. Die Eingabeeinheit 4 kann eine Tastatur oder ein mobiles Gerät sein, über welches Daten in den Computer 3 eingelesen werden können. Der Computer 3 umfasst ferner eine Recheneinheit 7, welche mit einem Datenspeicher 6 und einer Anzeigevorrichtung 5 sowie der Schnittstelle 21 gekoppelt ist. Mittels des Computers 3 lässt sich eine Reiseroutenplanung für ein Fahrzeug 1 durchführen, wie es später mit Bezug zu einem Ausführungsbeispiel des erfindungsgemäßen Verfahrens erläutert wird.

Das fahrzeuginterne Modul ist in einem Fahrzeug 1 untergebracht. Das Fahrzeug 1 umfasst einen Energiespeicher 2. Der Energiespeicher 2 kann von einer wiederaufladbaren Batterie gebildet sein. Diese Batterie liefert die Energie zum Antrieb des Fahrzeugs 1 und gegebenenfalls für weitere interne Verbraucher des Fahrzeugs 1. Bei dem Energiespeicher 2 kann es sich allerdings auch um einen Kraftstofftank handeln, welcher Kraftstoff zum Antrieb des Fahrzeugs 1 aufnimmt. Außerdem ist es möglich, dass es sich bei dem Fahrzeug 1 um ein Hybridfahrzeug handelt, welches sowohl mittels der Energie einer Batterie als auch mittels eines Kraftstoffs angetrieben werden kann.

Mit dem Energiespeicher 2 ist ein Sensor 15 zum Erfassen der Restenergiemenge in dem Energiespeicher 2 vorgesehen. Dieser Sensor erfasst den Ladezustand einer wiederaufladbaren Batterie oder den Füllstand des Kraftstoffs in einem Kraftstofftank.

Des Weiteren umfasst das Fahrzeug 1 eine weitere Recheneinheit 12, die mit dem Sensor 15, einer weiteren Anzeigevorrichtung 13 und einem weiteren Datenspeicher 14 datentechnisch gekoppelt ist.

Der Computer 3 und das Fahrzeug 1 sind ferner mit Funkschnittstellen 8 und 11 ausgestattet, die mit der Recheineinheit 7 bzw. der Recheneinheit 12 verbunden sind. Über eine Vermittlungsstation 11 kann auf diese Weise zwischen den Schnittstellen 8 und 11 eine Datenübertragung 18, 20 zwischen dem Computer 3 und dem Fahrzeug 1 hergestellt werden. Es können somit Daten insbesondere von dem Computer 3 an die Recheneinheit 12 des Fahrzeugs 1 übertragen werden. Bei der Funkverbindung kann es sich beispielsweise um eine Mobilfunkverbindung handeln. Es wäre jedoch auch möglich, dass es sich um eine drahtlose Netzwerkverbindung (WLAN) oder eine andere kurzreichweitige Funkverbindung handelt, welche einen Datenaustausch ermöglicht, wenn sich das Fahrzeug 1 in der Nähe des Computers 3 befindet. Wenn es sich bei dem Netzwerk um eine Mobilfunkverbindung handelt, ist es ferner möglich, dass der Computer 3 bzw. die Recheneinheit 12 des Fahrzeugs 1 Daten mit einem externen Server 16 austauschen, welcher auch über eine Funkschnittstelle 17 verfügt. Mittels des Servers 16 ist eine Datenübertragung 19 möglich, welche relevante Informationen für die Routenplanung enthalten, wie es später mit Bezug zu dem Ausführungsbeispiel des erfindungsgemäßen Verfahrens erläutert wird.

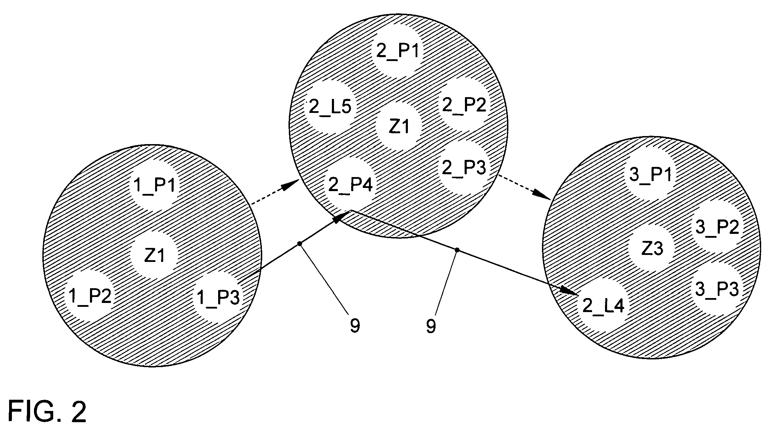

In Figur 2 ist schematisch ein Beispiel für eine zu planende Route dargestellt. Über einen elektronischen Terminkalender wurde der Recheneinheit 7 über die Schnittstelle 21 ein Terminplan für einen Tag des Nutzers übermittelt. Der Nutzer will an diesem Tag die Ziele Z1, Z2 und Z3 anfahren. Diesen Zielen Z1, Z2 und Z3 sind unterschiedliche geographische Positionen zugeordnet. Außerdem enthalten die Termindaten Zeitdaten, die angeben, wann der Nutzer die Ziele Z1, Z2 und Z3 erreichen will und wann er sie wieder verlassen will. Die zu planende Route sollte daher die Zielsequenz Z1, Z2 und Z3 so miteinander verbinden, dass der Nutzer die Termine bei diesen Zielen Z1, Z2, Z3 zu den zugeordneten Zeiten wahrnehmen kann. Dabei sollte außerdem sichergestellt sein, dass die Energiereserven im Energiespeicher 2 des Fahrzeugs 1 ausreichen, um die Ziele Z1, Z2 und Z3 anzufahren. Dabei ist es jedoch möglich, dass der Energiespeicher 2 bei Energieversorgungseinrichtungen während des Abfahrens der Routensequenz aufgefüllt wird.

Des Weiteren wird berücksichtigt, dass der Nutzer mit seinem Fahrzeug 1 die Ziele Z1, Z2 und Z3 nicht direkt anfahren kann, sondern er einen Standplatz für das Fahrzeug 1 benötigt. Der Datenspeicher 6 enthält daher eine Liste mit Standplätzen, deren geographische Positionen sowie die Information, ob es sich bei dem Standplatz um einen Parkplatz handelt oder der Standplatz eine Energieversorgungseinrichtung umfasst, mittels welcher der Energiespeicher 2 des Fahrzeugs 1 aufgefüllt werden kann.

Bei den in der Figur 2 dargestellten Zielen Z1, Z2 und Z3 sind dem Ziel Z1 die Parkplätze 1_P1, 1_P2, 1_P3 zugeordnet. Dem Ziel Z2 sind die Parkplätze 2_P1, 2_P2, 2_P3 und 2_P4 sowie die Energieversorgungseinrichtung 2_L5 zugeordnet. Dem dritten Ziel Z3 sind die Parkplätze 3_P1, 3_P2, 3_P3 und die Energieversorgungseinrichtung 3_L4 zugeordnet. Mittels der Recheneinheit 7 kann eine Routensequenz bestimmt werden, welche die Standplätze miteinander verbindet, welche den Zielen Z1, Z2 und Z3 zugeordnet sind, wie es durch die Pfeile 9 gezeigt ist.

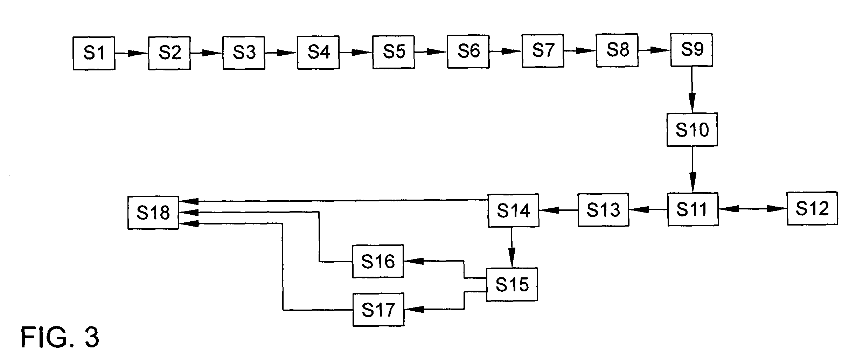

Ein Beispiel eines solchen Verfahrens zum Durchführen einer Reiseroutenplanung wird im Folgenden mit Bezug zur Figur 3 erläutert:
Zunächst werden der Rechnereinheit 7 des Computers 3 über die Schnittstelle 21 im Schritt S1 nutzerspezifische Nebenbedingungen übertragen. Diese Nebenbedingungen können Präferenzen des Nutzers angeben. Diese Präferenzen können beispielsweise die Standplätze für die Ziele betreffen. Der Nutzer kann eine maximale Entfernung eines Standplatzes von einem Ziel angeben. Ferner kann er eine bestimmte Präferenz für einen Parkplatztyp, zum Beispiel für einen Behindertenparkplatz, angeben. Des Weiteren kann der Nutzer angeben, mit welcher Priorität sichergestellt werden soll, dass immer während der geplanten Routensequenz eine bestimmte Restenergiemenge im Energiespeicher 2 des Fahrzeugs 2 gespeichert ist. Ferner kann der Nutzer angeben, wie wichtig es ihm ist, dass er rechtzeitig bei den Anfangszeiten der Termine am Ziel ankommt. Gegebenenfalls kann er hier eine gewisse Toleranz für Verspätungen angeben. Beispielsweise kann der Nutzer angeben, dass ein Ziel mit 5 Minuten Verspätung erreicht werden darf, wenn auf diese Weise verhindert wird, dass eine Energieversorgungseinrichtung zwischen zwei Zielen angesteuert werden muss, statt das Auffüllen des Energiespeichers 2 während eines Termins auf einem Standplatz bei einem Ziel durchzuführen.

Anschließend werden im Schritt S2 die Termindaten des Nutzers zum Beispiel von einem elektronischen Kalender auf einem mobilen Endgerät des Nutzers mittels der Schnittstelle 21 an die Recheneinheit 7 übertragen. Die Termindaten enthalten Informationen zu den geographischen Positionen von Zielen der zu planenden Reiseroute sowie zugehörigen Zeitdaten. Die Zeitdaten geben an, wann ein Termin bei einem bestimmten Ziel beginnt und wann er endet. Aus diesen Daten kann somit auch die Aufenthaltsdauer bei einem bestimmten Ziel ermittelt werden.

Im Schritt S3 zerlegt die Recheneinheit 7 die Termindaten in eine Zielsequenz mit aufeinanderfolgenden Zielen. Anschließend wird eine Routensequenz mit Routen berechnet, welche die Ziele der Zielsequenz miteinander verbindet. Dabei greift die Recheneinheit 7 auf ein Wegenetz, welches das Fahrzeug 1 befahren kann, zurück, welches in dem Datenspeicher 6 gespeichert ist. Im Schritt S4 werden hierfür verschiedene Routen bestimmt, die aufeinanderfolgende Ziele der Zielsequenz miteinander verbinden. Für jede Route erfolgt dann im Schritt S5 eine Abschätzung des Verkehrs beim Befahren der Route. Dabei kann die Recheneinheit 7 auf historische Verkehrsdaten zurückgreifen, welche in dem Datenspeicher 6 gespeichert sind. Außerdem können der Recheneinheit 7 Verkehrsdaten von dem externen Server 16 über die Funkverbindungen 19 und 18 übertragen werden. Ferner können auch Verkehrsdaten berücksichtigt werden, die aus einem anderen Fahrzeug heraus generiert worden sind, welches aktuell am Verkehrsgeschehen teilnimmt (sogenannte XFCD - extended floating car data). Des Weiteren können auch Verkehrsdaten berücksichtigt werden, die durch eine Fahrzeug-zu-Fahrzeug oder Fahrzeug-zu-X Kommunikation übertragen worden sind. Auf diese Weise kann die Recheneinheit 7 das Verkehrsaufkommen beim Befahren einer Route abschätzen.

Des Weiteren kann die Recheneinheit 7 im Schritt S6 die Geschwindigkeit des Fahrzeugs 1 beim Befahren einer Route abschätzen. Bei dieser Abschätzung kann die Recheneinheit den befahrenen Straßentyp berücksichtigen, der zusammen mit dem Wegenetz in dem Datenspeicher 6 gespeichert ist. Ferner kann die Recheneinheit 7 nutzerspezifische Daten zum Fahrverhalten des Fahrers, welcher die Routensequenz abfahren soll, berücksichtigen. Solche fahrerspezifischen Daten können auch in dem Datenspeicher 6 gespeichert sein. Sie können beispielsweise aus vergangenen Fahrten dieses Fahrers gewonnen werden.

Im Schritt S7 wird aus den Längen der verschiedenen Routen, welche die Ziele verbinden, der Abschätzung des Verkehrs beim Befahren der Route und den fahrerspezifischen Daten eine grobe Abschätzung durchgeführt, wie viel Zeit der Fahrer zum Befahren der einzelnen Routen benötigt. Dann werden alternative Routensequenzen ermittelt, welche möglichst wenig Zeit zum Erreichen der Ziele benötigen.

Nun wird im Schritt S8 von der Recheneinheit 7 die Restenergiemenge im Energiespeicher 2 für das Befahren der der Routensequenzen prognostiziert. Dabei werden der Energieverbrauch des Fahrzeugs 1 und Merkmale des Wegenetzes beim Befahren der Routensequenzen berücksichtigt. Beispielsweise kann berücksichtigt werden, wie die Steigung einer Straße des Wegenetzes ist, da sich die Steigung auf den Energieverbrauch des Fahrzeugs 1 auswirkt. Ferner können Verzögerungen bzw. Beschleunigungen auf Grund von Kurven berücksichtigt werden, da diese sich auch auf den Energieverbrauch des Fahrzeugs 1 auswirken. Des Weiteren wird in einem iterativen Verfahren die prognostizierte Restenergiemenge in dem Energiespeicher berücksichtigt. Der Ladezustand einer wiederaufladbaren Batterie oder die Kraftstoffmenge in einem Tank des Fahrzeugs 1 beeinflussen nämlich den Energieverbrauch des Fahrzeugs. Ist der Ladezustand einer Batterie niedriger, ergibt sich beim Befahren einer Route nämlich eine stärkere Änderung des Ladezustands als bei einem höheren Ladezustand der Batterie. Ferner werden interne Verbraucher des Fahrzeugs 1 bei dem prognostizierten Energieverbrauch berücksichtigt. Beispielsweise kann die Wahrscheinlichkeit dafür bestimmt werden, dass die Klimaanlage während des Befahrens der Route eingeschaltet ist. Ferner kann ein Nutzerverhalten des Fahrers des Fahrzeugs 1 berücksichtigt werden. Die Daten, aus denen der Energieverbrauch und damit die Restenergiemenge im Energiespeicher 2 für das Befahren der Routensequenzen prognostiziert werden, sind in dem Datenspeicher 6 gespeichert. Sie können von der Recheneinheit 7 ausgelesen werden und entsprechend bei der Prognose berücksichtigt werden.

Im Schritt S9 wählt die Recheneinheit 7 bevorzugte Routensequenzen aus. Dabei wird insbesondere berücksichtigt, ob die jeweilige Restenergiemenge bei den geographischen Positionen der Routensequenzen ausreicht, um die Ziele der Zielsequenz zu erreichen. Können nicht alle Ziele mit der Anfangsenergiemenge im Energiespeicher 2 des Fahrzeugs 1 erreicht werden, ist es erforderlich, dass der Energiespeicher 2 beim Befahren der Routensequenz aufgefüllt wird. In dem Datenspeicher 6 sind hierfür die geographischen Positionen von Energieversorgungseinrichtungen gespeichert. Die Recheneinheit 7 wählt bevorzugt solche Routensequenzen aus, bei denen der Energiespeicher 2 des Fahrzeugs 1 aufgefüllt werden kann, während sich das Fahrzeug 1 während eines Termins des Nutzers bei einem Standplatz befindet, der einem Ziel der Zielsequenz zugeordnet ist.

Für die Auswahl des Standplatzes wird im Schritt S10 für die Ziele der Zielsequenz ein Zielraum gebildet. Die Größe des Zielraums hängt von dem maximalen Abstand ab, den ein Standplatz von dem Ziel haben darf. Dieser maximale Abstand kann im Schritt S1 vom Nutzer eingegeben worden sein. In dem Datenspeicher 6 sind die geographischen Positionen aller Standplätze des Wegenetzes gespeichert. Es können nun die Standplätze ermittelt werden, die innerhalb des Zielraums liegen, der zu einem bestimmten Ziel gehört. Des Weiteren ist in dem Datenspeicher 6 gespeichert, ob es sich bei dem Standplatz um einen Parkplatz handelt oder ob der Standplatz eine Energieversorgungseinrichtung umfasst (siehe Figur 2).

Im Schritt S11 wird für jedes Ziel der Zielsequenz ein zugeordneter Standplatz bestimmt. Dabei werden folgende Faktoren berücksichtigt:
Aus der Prognose für die Restenergiemenge im Energiespeicher 2 des Fahrzeugs 1 wird bestimmt, ob es erforderlich ist, dass der Standplatz eine Energieversorgungseinrichtung umfasst. Falls dies der Fall ist, werden bei der folgenden Auswahl nur Standplätze mit Energieversorgungseinrichtungen berücksichtigt. Falls dies nicht der Fall ist, werden nur Parkplätze berücksichtigt.

Des Weiteren wird die Entfernung des Standplatzes von dem zugeordneten Ziel berücksichtigt sowie gegebenenfalls die Dauer, die der Nutzer benötigt, um von dem Standplatz zu dem Ziel zu gelangen. Dabei kann außerdem berücksichtigt werden, ob der Nutzer zu Fuß von dem Standplatz zum Ziel geht oder auf andere Weise von dem Standplatz zum Ziel gelangt. Aus der Entfernung und der Art des Erreichens des Ziels von dem Standplatz aus kann auf eine Dauer geschlossen werden, die ein Nutzer für den Weg vom Standplatz zum zugeordneten Ziel benötigt. Dabei können auch nutzerabhängige Gehgeschwindigkeiten berücksichtigt werden, die in dem Datenspeicher 6 gespeichert sind.

Des Weiteren wird die geographische Position des nächsten Ziels oder die geographische Position der Standplätze des nächsten Ziels oder eines ausgewählten Standplatzes des nächsten Ziels berücksichtigt. Alternativ oder zusätzlich kann die geographische Position des vorherigen Ziels oder die geographische Position der Standplätze des vorherigen Ziels oder eines ausgewählten Standplatzes des vorherigen Ziels berücksichtigt werden Diese Betrachtung kann dazu führen, dass nicht immer der zu einem Ziel nächste Standplatz der günstigste ist. Wenn durch diesen Standplatz die Route zum Erreichen des Ziels und des nächsten Ziels verlängert wird, kann ein anderer Standplatz aus zeitlicher Sicht günstiger sein, obwohl er weiter von dem zugeordneten Ziel entfernt ist.

Die zu den Zielen zugeordneten Standplätze werden von der Recheneinheit 7 nun so ausgewählt, dass sich weder zeitliche noch energetische Konflikte ergeben. Dies bedeutet, dass die Energiereserven im Energiespeicher des Fahrzeugs 1 beim Befahren der Routensequenz ausreichen, um zu den Standplätzen, die den Zielen zugeordnet sind, zu gelangen, wobei berücksichtigt wird, dass der Energiespeicher 2 bei Standplätzen mit Energieversorgungseinrichtungen teilweise oder vollständig aufgefüllt werden kann. Ferner wird sichergestellt, dass die Ziele rechtzeitig, d.h. in Übereinstimmung mit den Termindaten, vom Nutzer erreicht werden, wobei nicht nur die Zeit zum Befahren der Routen zwischen den Standplätzen berücksichtigt wird, sondern auch die Dauer, die ein Nutzer benötigt, um von dem Standplatz zu dem zugeordneten Ziel zu gelangen.

Beim Schritt S12 kann außerdem die Verfügbarkeitswahrscheinlichkeit der Standplätze im Zielraum zu den Zielen ermittelt werden. Hierfür kann einem Schritt S11 die Ankunftszeit des Fahrzeugs am zu untersuchenden Standplatz und die Aufenthaltsdauer an diesem Standplatz ermittelt und mit historischen Daten, die in dem Datenspeicher 6 gespeichert sind, verglichen werden. Des Weiteren können über die Datenverbindungen 18 und 19 bereits getätigte Buchungen Dritter für die Standplätze von dem externen Server 16 abgerufen werden. Liegen bereits für die gewünschte Zeit Buchungen vor, ist die Verfügbarkeitswahrscheinlichkeit des entsprechenden Standplatzes sehr gering bzw. Null. Solche Standplätze werden dann im Schritt S11 von der Recheineinheit 7 für die Routensequenz nicht berücksichtigt.

Anschließend wird im Schritt S13 eine Feinplanung der Routensequenz durchgeführt. Dabei werden die Routen zwischen zwei Standplätzen, die zu aufeinanderfolgenden Zielen der Zielsequenz gehören, nochmals in zeitlicher und energetischer Hinsicht optimiert.

Danach wird im Schritt S14 eine Optimierung der Routen im Tagesablauf durchgeführt, wobei insbesondere sichergestellt wird, dass die Routensequenz in zeitlicher Hinsicht zu den gesuchten Termindaten passt. Es wird sichergestellt, dass der Nutzer zu den Anfangszeiten der Termine bei den entsprechenden geographischen Positionen ankommt. Dabei wird auch die nutzbare Zeit des Nutzers optimiert. Dies bedeutet insbesondere, dass die Energiemenge in dem Energiespeicher 2 des Fahrzeugs 1 während einer Standzeit des Fahrzeugs 1 während eines Termins des Nutzers erhöht wird. Wenn die Recheneinheit 7 eine Routensequenz berechnen konnte, die keine zeitlichen und energetischen Konflikte mit den Termindaten verursacht, kann die berechnete Routensequenz im Schritt S18 über die Anzeigevorrichtung 5 angezeigt oder über Funkschnittstelle 8 ausgegeben werden. Die Details der Visualisierung der Routensequenz werden später erläutert.

Wenn sich hingegen herausstellt, dass mittels der Recheneinheit 7 keine Routensequenz berechenbar ist, die in zeitlicher Hinsicht keine Konflikte mit den Termindaten verursacht, werden von der Recheneinheit 7 im Schritt S15 verschiedene alternative Routensequenzen geprüft. Dabei bleiben zunächst die energetischen und geographischen Nebenbedingungen unverändert, d.h. es soll weiterhin die den Termindaten entsprechende Zielsequenz von der Routensequenz durchfahren werden. Ferner wird von der Recheneinheit 7 sichergestellt, dass die prognostizierte Energiemenge im Energiespeicher 2 des Fahrzeugs 1 ausreicht, um alle Ziele der Zielsequenz zu erreichen, wobei berücksichtigt wird, dass der Energiespeicher 2 beim Befahren der Routensequenz zwischenzeitlich durch Energieversorgungseinrichtungen aufgefüllt werden kann. Dabei wird eine Routensequenz berechnet, die in zeitlicher Hinsicht so nah wie möglich an die gewünschten Termindaten herankommt.

Im Schritt S16 werden dann angepasste Termindaten ausgegeben, wobei es eine zugeordnete Routensequenz gibt, welche mit den angepassten Termindaten keine Konflikte erzeugt. Mittels der Eingabeeinheit 4 kann der Nutzer die angepassten Termindaten annehmen. Sie werden dann über die Schnittstelle 21 an den elektronischen Kalender übertragen. Gegebenenfalls werden automatisch Nachrichten für weitere Teilnehmer des Termins erzeugt, um diese von den angepassten Termindaten zu unterrichten.

Falls es nicht möglich ist, eine Routensequenz ohne zeitliche oder energetische Konflikte zu berechnen, die auf angepassten Termindaten beruht, wird im Schritt S17 eine Routensequenz berechnet, bei welcher ein Ziel oder mehrere Ziele gestrichen wurden. Diese Änderungen der Termindaten sowie die angepasste Routensequenz werden ausgegeben. Wenn der Nutzer die angepasste Routensequenz und die geänderten Termindaten annimmt, wird die angepasste Routensequenz als aktuelle Routensequenz gespeichert und die geänderten Termindaten werden an den elektronischen Kalender des Nutzers übertragen.

Sobald die Routensequenz für die Reiseroutenplanung feststeht, wird im Schritt S18 die berechnete Routensequenz angezeigt und ausgegeben. Ferner werden von der Recheneinheit 7 über die Datenverbindungen 18 und 19 bei dem externen Server 16 die gewünschten Standplätze der Routensequenz reserviert.

Gemäß einer Weiterbildung des Ausführungsbeispiels des erfindungsgemäßen Verfahrens werden noch verschiedene Unsicherheiten, die sich bei der Reiseroutenplanung ergeben, bestimmt und miteinander gekoppelt. Zum einen kann eine Unsicherheit dafür bestimmt werden, dass der Fahrer die prognostizierte Ankunftszeit bei einem Standplatz zu einem bestimmten Zeitpunkt erreicht. Ferner kann die Wahrscheinlichkeit dafür berücksichtigt werden, dass der Fahrer den Standplatz bei der prognostizierten Abfahrtszeit auch tatsächlich verlässt. Die Wahrscheinlichkeiten können anhand von Kurven dargestellt werden, die unterschiedliche Höhen, d.h. Wahrscheinlichkeiten, angeben und die unterschiedliche Breiten, d.h. unterschiedliche Abweichungen von dem prognostizierten Wert, haben.

Des Weiteren kann eine Abweichungswahrscheinlichkeit vom nominalen Verkehrsfluss berücksichtigt werden. In Kombination mit der Unsicherheit, dass der Fahrer einen bestimmten Standplatz zur prognostizierten Zeit erreicht bzw. wieder verlässt, können Wahrscheinlichkeiten für verkehrsbedingte Ankunfts- und Abfahrtszeiten berechnet werden.

Ferner kann eine Unsicherheit berücksichtigt werden, welche die Belegung einer Energieversorgungseinrichtung oder eines Parkplatzes betrifft. Gegebenenfalls muss das Fahrzeug 1 eine gewisse Zeit warten, bis der Parkplatz bzw. die Energieversorgungseinrichtung frei wird. Hierdurch können die Wahrscheinlichkeiten für die Ankunfts- und Abfahrtszeiten weiter modifiziert werden.

Die Kombinationen der Wahrscheinlichkeitsverteilungen können von den Recheneinheiten 7 und 12 zur Optimierung der Vorausplanung der Routensequenz bzw. der Optimierung der Routensequenz während der Fahrt genutzt werden. Dabei wird die Vorhersagbarkeit und Planbarkeit der Reiseroute in dem Netzwerk maximiert.

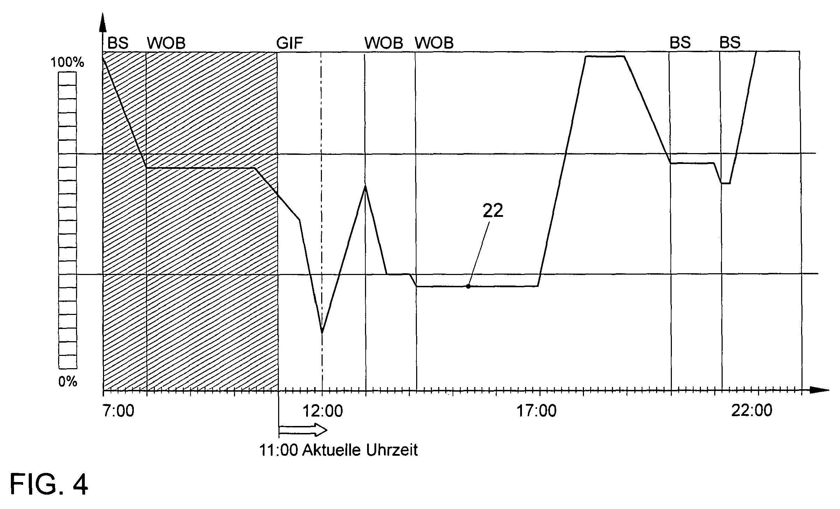

Im Folgenden wird mit Bezug zu den Figuren 4 bis 12 beschrieben, wie die Ausgabe der von dem vorstehend beschriebenen Verfahren berechneten Reiseroutenplanung erfolgt:
In Figur 4 ist die prognostizierte Restenergiemenge für die Routensequenz dargestellt, wie sie dem Nutzer angezeigt werden kann. Es wird ein Koordinatensystem angezeigt, auf dessen horizontaler Achse die Zeit aufgetragen ist, und auf dessen vertikaler Achse die Restenergiemenge im Energiespeicher 2 des Fahrzeugs von 0% bis 100% aufgetragen ist. Auf der horizontalen Achse sind außerdem Abkürzungen für Orte angegeben, die zu den jeweiligen Zeiten bei der Routensequenz erreicht werden. Aus der dargestellten Kurve 22 ist beispielsweise ersichtlich, dass das Fahrzeug um 7.00 Uhr von Braunschweig Richtung Wolfsburg fährt. In Wolfsburg ergibt sich eine Standzeit des Fahrzeugs. Um 12.00 Uhr wird dann der Energiespeicher 2 des Fahrzeugs 1 aufgefüllt. Um 17.00 Uhr und kurz vor 22.00 Uhr wird der Energiespeicher 2 nochmals aufgefüllt. Außerdem ist aus der Kurve 22 ersichtlich, dass, wenn der Energiespeicher 2 um 12.00 Uhr nicht aufgefüllt worden wäre, die Energiereserven während des Befahrens einer Route der Routensequenz aufgebraucht worden wären. Es hätte sich somit ein energetischer Konflikt ergeben, der dazu geführt hätte, dass der Fahrer mit dem Fahrzeug liegengeblieben wäre.

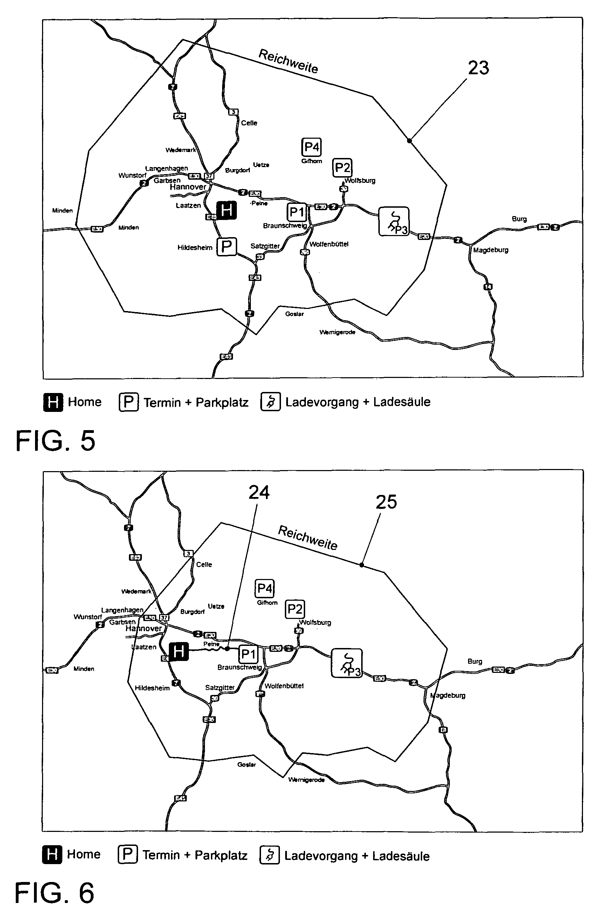

Des Weiteren kann, wie in Figur 5 gezeigt, nach der Berechnung der Routensequenz mithilfe des in dem Datenspeicher 6 gespeicherten Wegenetzes eine graphische Kartendarstellung erzeugt werden, bei welcher die geographischen Positionen der zu den Zielen.gehörigen Standplätze mittels der Zeichen P1 bis P4 dargestellt werden. Umfasst ein Standplatz eine Energieversorgungseinrichtung, wird zusätzlich ein Zeichen dargestellt, welches darauf hinweist, dass zum Beispiel die Batterie des Elektrofahrzeugs aufgeladen werden kann. Des Weiteren wird die geographische Position einer Referenzposition H angezeigt. Die Referenzposition kann beispielsweise der Wohnsitz des Nutzers oder seine Arbeitsstätte sein.

Geht man davon aus, dass der Nutzer die Routensequenz bei der Referenzposition H startet, wird auf Basis der Ausgangsenergiemenge im Energiespeicher 2 des Fahrzeugs und auf Basis des gespeicherten Wegenetzes bestimmt, welche Punkte des Wegenetzes von der Referenzposition H aus noch erreichbar sind. Bei dieser Reichweite wird auch der prognostizierte Energieverbrauch beim Befahren des Wegenetzes berücksichtigt. In der graphischen Kartendarstellung wird nun eine Grenzlinie 23 dargestellt, welche die Reichweite des Fahrzeugs 2 bei der geographischen Referenzposition H visualisiert. Die Grenzlinie 23 grenzt die Fläche mit den Punkten des Wegenetzes ab, die von der Position des Fahrzeugs 2 aus mit der Restenergiemenge des Energiespeichers 2 erreichbar sind.

Bei einer weiteren in Figur 6 wiedergegebenen Darstellung wird zum einen die Route 24 von der geographischen Referenzposition H zum Standplatz P1, welcher dem ersten Ziel zugeordnet ist, dargestellt. Ferner wird die Restenergiemenge des Energiespeichers 2 des Fahrzeugs 1 bei der geographischen Position des Standplatzes P1 wie vorstehend beschrieben prognostiziert. Für diese Restenergiemenge wird erneut die Reichweite des Fahrzeugs 1 bestimmt, d.h. es werden die Punkte des Wegenetzes ermittelt, die von dem Standplatz P1 aus mit der prognostizierten Restenergiemenge noch erreichbar sind. Für diesen Reichweitenbereich wird erneut eine Grenzlinie 25 auf der graphischen Kartendarstellung angezeigt.

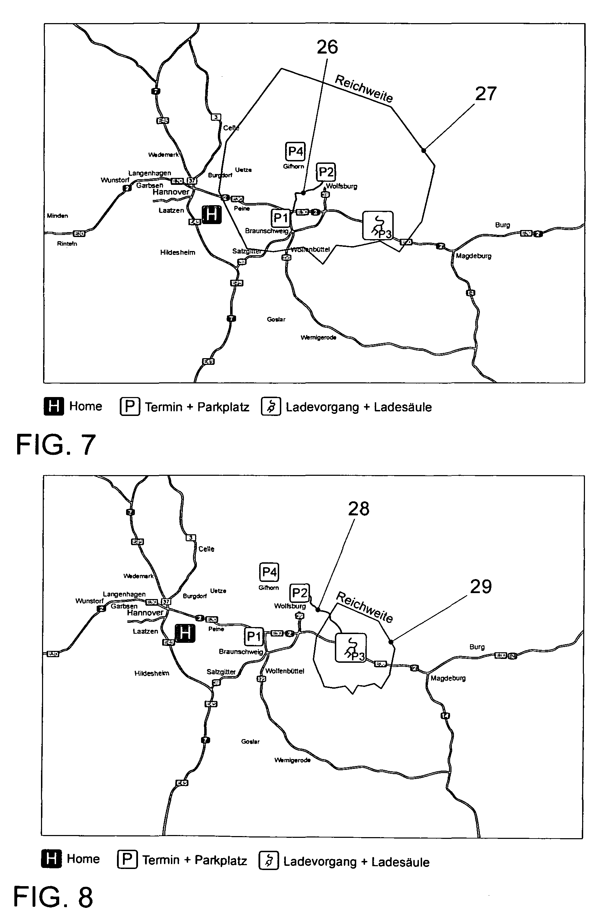

In Figur 7 ist die Route 26 von dem Standplatz P1 des ersten Ziels zu dem Standplatz P2 des zweiten Ziels in der graphischen Kartendarstellung angezeigt. Des Weiteren wird wie bei der Visualisierung gemäß Figur 6 die Restreichweite des Fahrzeugs 2 mit der prognostizierten Restenergiemenge beim Standplatz P2 ermittelt und anhand der Grenzlinie 27 dargestellt.

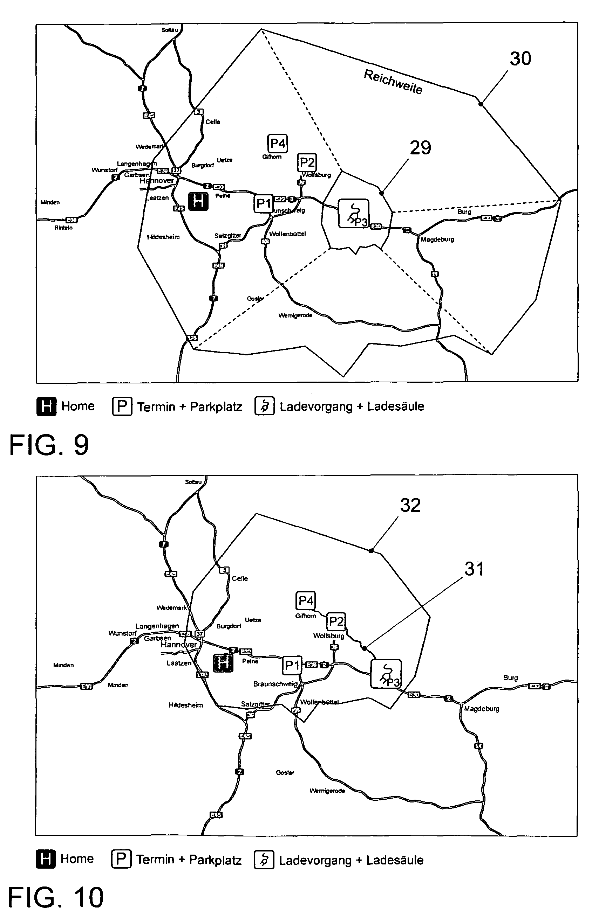

Gleichermaßen wird bei der Darstellung gemäß Figur 8 die Route 28 von dem Standplatz P2 des zweiten Ziels zu dem Standplatz P3 des dritten Ziels auf der graphischen Kartendarstellung angezeigt. Ferner wird eine Grenzlinie 29 für die Restreichweite beim Standplatz P3 dargestellt. Wie aus Figur 8 ersichtlich, reicht diese Restreichweite nicht mehr dafür aus, dass das Fahrzeug 2 den Standplatz P4 des vierten Ziels erreicht. Die Routensequenz wurde jedoch im Voraus so berechnet, dass beim dritten Ziel ein Standplatz ausgewählt wurde, welcher eine Energieversorgungseinrichtung umfasst. Beim Standplatz P3 kann der Energiespeicher 2 des Fahrzeugs 1 somit aufgefüllt werden. Wie in Figur 9 gezeigt, wird die Restreichweite des Fahrzeugs 1 beim Standplatz P3 anhand der Grenzlinie 29 vor dem Auffüllen des Energiespeichers 2 angezeigt und anhand der Grenzlinie 30 nach dem Auffüllen des Energiespeichers. Wie aus Figur 9 ersichtlich, reicht die Restreichweite nach dem Auffüllen des Energiespeichers 2 aus, um den Standplatz P4 für das nächste Ziel zu erreichen. Außerdem reicht die Restreichweite aus, um die Referenzposition H zu erreichen.

In Figur 10 ist schließlich die Route 31 von dem Standplatz P3 zu dem Standplatz P4 des vierten Ziels auf der graphischen Kartendarstellung gezeigt. Ferner ist anhand der Grenzlinie 32 die Restreichweite des Fahrzeugs 1 beim Standplatz P4 gezeigt. Es ist insbesondere ersichtlich, dass die Referenzposition H weiterhin innerhalb dieser Restreichweite liegt. Es ist bei der Routensequenz somit sichergestellt worden, dass die Referenzposition H immer erreicht werden kann.

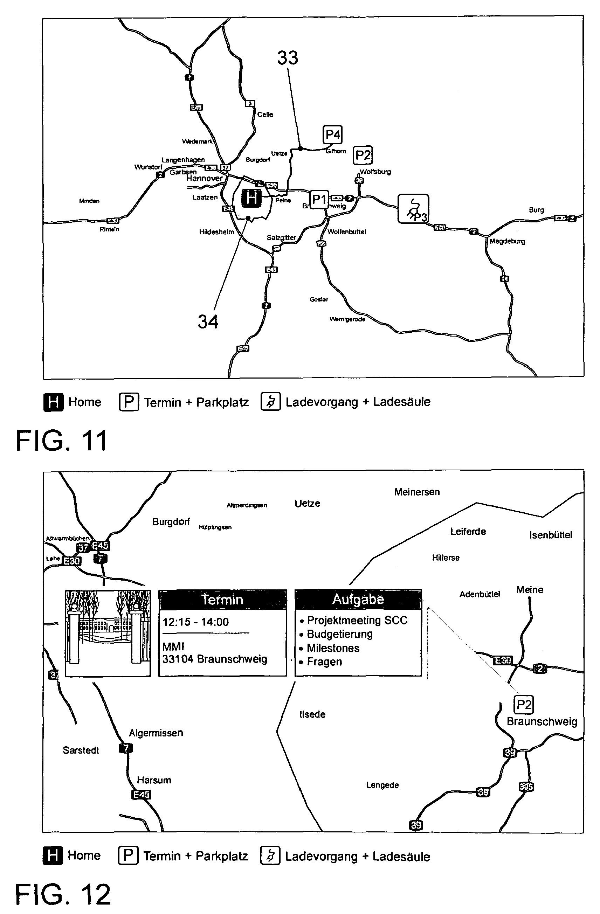

In Figur 11 ist abschließend die Route 33 auf der graphischen Kartendarstellung gezeigt, welche von dem Standplatz P4 zurück zu der Referenzposition H führt. Ferner ist die Restreichweite bei der Referenzposition H am Ende der Routensequenz anhand der Grenzlinie 34 dargestellt. Am Ende der Routensequenz ergibt sich somit eine relativ geringe Restenergiemenge. Der Energiespeicher 2 des Fahrzeugs soll daher bei der Referenzposition H wieder aufgefüllt werden.

In Figur 12 sind noch Zusatzinformationen zu einem Standplatz P2 dargestellt. Diese Zusatzinformationen können vom Nutzer durch Betätigung der Eingabeeinheit 4 vor Fahrtantritt oder - wie es später erläutert wird - auch während der Fahrt abgerufen werden.

Mit Bezug zur Figur 3 wurde ein Beispiel für das Verfahren zum Durchführen einer Reiseroutenplanung für ein Fahrzeug beschrieben. Nachdem die Reiseroutenplanung abgeschlossen ist und eine Routensequenz vorliegt, wird diese während des Befahrens der Routensequenz dazu verwendet, dem Nutzer im Fahrzeug eine Routenführung anzubieten und die Routensequenz gegebenenfalls anzupassen. Ein Beispiel für diese weiteren Verfahrensschritte wird im Folgenden erläutert:
Über die Datenverbindungen 18 und 20 werden zunächst Daten zu der von der Recheneinheit 7 ermittelten Routensequenz an die Recheneinheit 12 im Fahrzeug 1 übertragen. Die Recheinheit 12 ist im Wesentlichen wie die Recheneinheit 7 ausgebildet. Ferner umfasst auch der Datenspeicher 14 im Fahrzeug 1 dieselben Informationen wie der Datenspeicher 6 des Computers 3. Falls erforderlich, können die Datenspeicher 6 und 14 auch über die Datenverbindungen 18, 20 synchronisiert werden.

Während der Fahrt werden dem Fahrer des Fahrzeugs 1 nun Navigationsinformationen zum Befahren der Routensequenz über die Anzeigevorrichtung 13 ausgegeben. Die Recheneinheit 12 stellt somit in Verbindung mit der Anzeigevorrichtung 13 ein herkömmliches Fahrzeugnavigationssystem bereit. Hierfür ist die Recheneinheit 12 auch mit einem Empfänger für Satellitensignale, zum Beispiel einem GPS-Empfänger gekoppelt. Dieser Empfänger kann auch in der Recheneinheit 12 integriert sein.

Gleichzeitig werden der Recheneinheit 12 fortwährend Daten des Sensors 15 zu der Restenergiemenge im Energiespeicher 2 übermittelt. Wenn die Abweichung der erfassten Restenergiemenge im Energiespeicher 2 des Fahrzeugs 1 von der prognostizierten Restenergiemenge bei der Berechnung mittels der Recheneinheit 7 einen bestimmten Grenzwert, z.B. 10% der prognostizierten Restenergiemenge, überschreitet, kann die Routensequenz auf Basis der erfassten Restenergiemenge neu berechnet werden. Wenn die erste Restenergiemenge geringer als die prognostizierte Restenergiemenge ist, wird insbesondere geprüft, ob die Restenergiemenge ausreicht, um alle Ziele und Energieversorgungseinrichtungen noch zu erreichen. Wenn dies nicht der Fall ist, wird die Routensequenz von der Recheneinheit 12, wie es mit Bezug zur Figur 3 erläutert wurde, neu berechnet und ausgegeben.

Des Weiteren wird die gegenwärtige Position mit der prognostizierten Position zu einem bestimmten Zeitpunkt verglichen. Ergibt sich in zeitlicher Hinsicht eine Abweichung, insbesondere wenn zu einem bestimmten Zeitpunkt eine geographische Position der Routensequenz noch nicht erreicht wurde, wird geprüft, ob die Ziele der Routensequenz zu den zughörigen Zeitdaten der Termindaten weiterhin erreicht werden können. Dabei wird auch die prognostizierte Restenergiemenge in dem Energiespeicher des Fahrzeugs für vorausliegende Abschnitte der Routensequenz berücksichtigt. Wenn es beispielsweise aufgrund des tatsächlichen Energieverbrauchs erforderlich ist, einen ungeplanten Zwischenstopp oder mehrere ungeplante Zwischenstopps für das Auffüllen der Energiemenge in dem Energiespeicher 2 einzulegen, wird die zusätzliche Zeit, die für diesen Zwischenstopp erforderlich ist, mit berücksichtigt.

Des Weiteren können aktuelle Verkehrsdaten berücksichtigt werden, die der Recheneinheit 12 über die Datenverbindungen 19 und 20 von einem externen Server 16 übermittelt werden. In Abhängigkeit von den aktuellen Verkehrsdaten kann eine aktualisierte Zeit zum Erreichen des nächsten Ziels berechnet werden. Ferner kann die Routensequenz in zeitlicher Hinsicht aktualisiert werden. Auch in diesem Fall wird geprüft, ob die Ziele der Routensequenz zu den zugehörigen Zeitdaten der Termindaten noch erreicht werden können.

Wenn sich ergibt, dass bestimmte Termine in zeitlicher Hinsicht nicht eingehalten werden können, wird eine Ausgabe für den Fahrer erzeugt, damit dieser die Termine gegebenenfalls entsprechend anpassen kann und weitere Teilnehmer an den Terminen informieren kann.

Die Recheneinheit 12 berechnet in diesem Fall eine angepasste Routensequenz, bei der die Zeitdaten der Termindaten angepasst wurden oder bestimmte Termine gestrichen wurden, wie dies vorstehend bereits erläutert wurde.

Des Weiteren können die Verfügbarkeitswahrscheinlichkeiten für die Verfügbarkeit der Standplätze der Routensequenz aktualisiert werden. Entsprechende Daten können der Recheneinheit 12 beispeisweise über die Datenverbindungen 19, 20 von dem externen Server 16 übertragen werden. Wenn sich beispielsweise ergibt, dass ein bestimmter Parkplatz nicht mehr frei ist, passt die Recheneinheit 12 die Routensequenz so an, dass ein anderer Parkplatz für ein bestimmtes Ziel bei der Routensequenz ausgewählt wird. Wenn sich ergibt, dass ein Standplatz mit einer Energieversorgungseinrichtung zu der gewünschten Zeit der Routensequenz nicht frei ist, kann die Recheneinheit 12 einen anderen Standplatz mit einer Energieversorgungseinrichtung für eine angepasste Routensequenz auswählen. Dieser andere Standplatz mit der Energieversorgungseinrichtung liegt dabei möglicherweise bei einem anderen Ziel. Bei der Optimierung der Routensequenz maximiert die Recheneinheit 12 die für den Nutzer verfügbare Zeit. Etwaige Wartezeiten während des Auffüllens der Energiemenge des Energiespeichers 2 werden dabei minimiert und nach Möglichkeit in Zeiten gelegt, zu denen der Nutzer einen Termin wahrnimmt.

Bei allen Anpassungen der Routensequenz während der Fahrt werden auch angepasste graphische Kartendarstellungen und Diagramme, wie sie in den Figuren 4 bis 12 gezeigt sind, erzeugt und bei Bedarf über die Anzeigevorrichtung 13 im Fahrzeug 1 ausgegeben.

Durch die erfindungsgemäße Reiseroutenplanung kann eine ganzheitliche Berechnung einer Fahraufgabe mit einer Zielsequenz und entsprechenden örtlichen, zeitlichen und energetischen Restriktionen berechnet werden. Dabei wird insbesondere bei der Planung zum Auffüllen der Energiereserven des Fahrzeugs 1 eine gekoppelte Betrachtung aller Ziele der Zielsequenz vorgenommen. Diese ist insbesondere dann wichtig, wenn es sich bei Fahrzeug 1 um ein Elektrofahrzeug mit einer begrenzten Reichweite handelt. Ferner werden nicht nur die Fahrtrouten für das Fahrzeug 1 betrachtet, sondern auch die Wege von Standplätzen des Fahrzeugs 1 zu den gewünschten Zielen. Die Dauer für diese Wege werden in die Berechnung der Routensequenz und insbesondere in die Auswahl der Standplätze für die Ziele einbezogen.

### Bezugszeichenliste

- 1: Fahrzeug
- 2: Energiespeicher
- 3: Computer
- 4: Eingabeeinheit
- 5: Anzeigevorrichtung
- 6: Datenspeicher
- 7: Recheneinheit
- 8: Funkschnittstelle
- 9: Pfeile
- 10: Vermittlungsstation
- 11: Vermittlungsstation
- 12: Recheneinheit
- 13: Anzeigevorrichtung
- 14: Datenspeicher
- 15: Sensor
- 16: externer Server
- 17: Funkschnittstelle
- 18, 19, 20: Datenverbindungen
- 22: Energiekurve der Routensequenz
- 23: Grenzlinie
- 24: Route
- 25: Grenzlinie
- 26: Route
- 27: Grenzlinie
- 28: Route
- 29: Grenzlinie
- 30: Grenzlinie
- 31: Route
- 32: Grenzlinie
- 33: Route
- 34: Grenzlinie

## Patentansprüche

1. Verfahren zum Durchführen einer Reiseroutenplanung für ein Fahrzeug (1), das einen Energiespeicher (2) zum Speichern der Energie zum Antrieb des Fahrzeugs (1) umfasst, bei dem
- Termindaten, welche geographische Positionen von Zielen der zu planenden Reiseroute und zugehörige Zeitdaten umfassen, an eine Recheneinheit (7) übermittelt werden, die mit einem Datenspeicher (6) gekoppelt ist, in dem Daten zu einem Wegenetz für das Fahrzeug (1) und Daten zu den geographischen Positionen von Energieversorgungseinrichtungen gespeichert sind,
- die Recheneinheit (7) prüft, ob eine Routensequenz berechenbar ist, welche die geographischen Positionen der zu den Termindaten gehörigen Ziele so verbindet, dass die Ziele zu den zugehörigen Zeitdaten der Termindaten erreicht werden,
- falls mittels der Recheneinheit (7) keine solche Routensequenz berechenbar ist, sie angepasste Termindaten bestimmt, für welche eine solche Routensequenz berechenbar ist, und
- die angepassten Termindaten ausgegeben werden,
**dadurch gekennzeichnet,**
- **dass** bei der Prüfung, ob die Routensequenz berechenbar ist, die prognostizierte Restenergiemenge in dem Energiespeicher (2) des Fahrzeugs (1) für das Befahren der Routensequenz bestimmt und berücksichtigt wird, und
- **dass** die Recheneinheit (7) bei der Berechnung der Routensequenz die für den Nutzer des Fahrzeugs (1) nutzbare Zeit optimiert, wobei berücksichtigt wird, dass die Energiemenge in dem Energiespeicher (2) des Fahrzeugs (1) von einer Energieversorgungseinrichtung während einer Standzeit des Fahrzeugs (1) während eines Termins des Nutzers erhöht werden kann.

2. Verfahren nach Anspruch 1, **dadurch gekennzeichnet, dass** bei den angepassten Termindaten geographischen Positionen von Zielen neue Zeitdaten zugeordnet werden.

3. Verfahren nach Anspruch 1 oder 2, **dadurch gekennzeichnet, dass** bei den angepassten Termindaten Ziele gestrichen sind.

4. Verfahren nach einem der vorhergehenden Ansprüche, **dadurch gekennzeichnet, dass** die Recheneinheit (7) die prognostizierte Restenergiemenge in dem Energiespeicher (2) anhand des prognostizierten Energieverbrauchs für das Befahren der Routensequenz berechnet, wobei der prognostizierte Energieverbrauch zum Antrieb des Fahrzeugs (1) und der prognostizierte Energieverbrauch interner Verbraucher des Fahrzeugs (1) berücksichtigt werden.

5. Verfahren nach einem der vorhergehenden Ansprüche, **dadurch gekennzeichnet, dass**
- in dem Datenspeicher (6) ferner Daten zu geographischen Positionen von Standplätzen, die Parkplätze oder Energieversorgungseinrichtungen umfassen, für das Fahrzeug (1) für die Ziele der Zielsequenz gespeichert sind,
- die Recheneinheit (7) bei der Berechnung einer Routensequenz ferner für die Ziele der Zielsequenz jeweils zu den Zielen zugeordnete Standplätze in der Nähe des jeweiligen Ziels ermittelt, für jedes Ziel der Zielsequenz einen zugeordneten Standplatz bestimmt, wobei bei der Bestimmung die Entfernung des Standplatzes von dem zugeordneten Ziel, die geographische Position des nächsten Ziels oder die geographischen Positionen der Standplätze des nächsten Ziels und/oder die prognostizierte Restenergiemenge in dem Energiespeicher (2) für das Befahren der Routensequenz berücksichtigt werden, und die Routensequenz sich aus Routen zwischen Standplätzen aufeinanderfolgender Ziele der Zielsequenz zusammensetzt, und
- bei der Prüfung, ob eine Routensequenz die geographischen Positionen der zu den Termindaten gehörigen Ziele so verbindet, dass die Ziele zu den zugehörigen Zeitdaten der Termindaten erreicht werden, eine Dauer berücksichtigt wird, die ein Nutzer benötigt, um von dem Standplatz, welcher einen Ziel zugeordnet ist, zu diesem Ziel zu gelangen.

6. Verfahren nach Anspruch 5, **dadurch gekennzeichnet, dass** während des Befahrens der berechneten Routensequenz die Restenergiemenge im Energiespeicher (2) des Fahrzeugs (1) erfasst wird und mit der prognostizierten Restenergiemenge des Fahrzeugs (1) für eine entsprechende Routenposition verglichen wird und, falls die Abweichung der erfassten Restenergiemenge von der prognostizierten Restenergiemenge einen Grenzwert überschreitet, geprüft wird, ob die Ziele der Routensequenz zu den zugehörigen Zeitdaten der Termindaten weiterhin erreicht werden, wobei die prognostizierte Restenergiemenge in dem Energiespeicher (2) des Fahrzeugs (1) für das Befahren der Routensequenz bestimmt wird.

7. Verfahren nach Anspruch 6 oder 7, **dadurch gekennzeichnet, dass**, falls die Prüfung ergibt, dass die Ziele der Routen nicht zu den zugehörigen Zeitdaten erreicht werden können, eine angepasste Routensequenz berechnet wird oder angepasste Termindaten bestimmt werden und die angepasste Routensequenz oder die angepassten Termindaten ausgegeben werden.

8. Vorrichtung zur Reiseroutenplanung für ein Fahrzeug (1), das einen Energiespeicher (2) zum Speichern der Energie zum Antrieb des Fahrzeugs (1) umfasst, mit
- einer Recheneinheit (7),
- einem mit der Recheneinheit (7) gekoppelten Datenspeicher (6), in dem Daten zu einem Wegenetz und Daten zu geographischen Positionen von Energieversorgungseinrichtungen für das Fahrzeug (1) gespeichert sind,
- einer mit der Recheneinheit (7) gekoppelten Schnittstelle (21), über welche Termindaten, welche geographische Positionen von Zielen der zu planenden Reiseroute und zugehörige Zeitdaten umfassen, an die Recheneinheit (7) übermittelbar sind,
- einer mit der Recheneinheit (7) gekoppelten Ausgabeeinheit (5), mittels welcher eine von der Recheneinheit (7) berechnete Routensequenz und/oder angepasste Termindaten ausgebbar sind und mittels welcher prüfbar ist, ob eine Routensequenz berechenbar ist, welche die geographischen Positionen der zu den Termindaten gehörigen Ziele so verbindet, dass die Ziele zu den zugehörigen Zeitdaten der Termindaten erreicht werden und, falls keine solche Routensequenz berechenbar ist, mit der Recheneinheit (7) angepasste Termindaten bestimmbar sind, für welche eine solche Routensequenz berechenbar ist,
**dadurch gekennzeichnet, dass**
- mittels der Recheneinheit (7) die prognostizierte Restenergiemenge in dem Energiespeicher (2) des Fahrzeugs (1) für das Befahren der Routensequenz bestimmt und berücksichtigt wird und bei der Berechnung der Routensequenz die für den Nutzer des Fahrzeugs (1) nutzbare Zeit optimierbar ist, wobei berücksichtigt wird, dass die Energiemenge in dem Energiespeicher (2) des Fahrzeugs (1) von einer Energieversorgungseinrichtung während einer Standzeit des Fahrzeugs (1) während eines Termins des Nutzers erhöht werden kann.

9. Vorrichtung nach Anspruch 8, **dadurch gekennzeichnet, dass**
- die Vorrichtung ein fahrzeugexternes Modul (3) und ein fahrzeuginternes Modul aufweist, wobei das fahrzeugexterne Modul (3) die Recheneinheit (7) umfasst,
- das fahrzeugexterne (3) und das fahrzeuginterne Modul über eine Schnittstelle (8, 11) zumindest zeitweise datentechnisch miteinander gekoppelt sind, so dass zumindest eine Routensequenz von dem fahrzeugexternen Modul (3) an das fahrzeuginterne Modul übertragbar ist,
- dass fahrzeuginterne Modul eine weitere Recheneinheit (12), eine weitere Ausgabeeinheit (13), einen Speicher (14) zum Speichern einer von dem fahrzeugexternen Modul (3) übertragenen Routensequenz und einen Sensor (15) zum Erfassen der Restenergiemenge im Energiespeicher (2) des Fahrzeugs (1) umfasst,
- mittels der weiteren Recheneinheit (12) während des Befahrens der berechneten Routensequenz die erfasste Restenergiemenge im Energiespeicher (2) des Fahrzeugs (1) mit der prognostizierten Restenergiemenge des Fahrzeugs (1) für eine entsprechende Routenposition der in dem Speicher (14) gespeicherten Routensequenz vergleichbar ist und, falls die Abweichung der erfassten Restenergiemenge von der prognostizierten Restenergiemenge einen Grenzwert überschreitet, mittels der weiteren Recheneinheit (12) prüfbar ist, ob die Ziele der Routensequenz zu den zugehörigen Zeitdaten der Termindaten weiterhin erreicht werden, wobei die prognostizierte Restenergiemenge in dem Energiespeicher (2) des Fahrzeugs (1) für das Befahren der Routensequenz bestimmt wird, und,
- falls die Prüfung ergibt, dass die Ziele der Routensequenz nicht zu den zugehörigen Zeitdaten erreicht werden können, die weitere Recheneinheit (12) zur Ausgabe über die weitere Ausgabeeinheit (13) eine angepasste Routensequenz berechnet oder angepasste Termindaten bestimmt.

## Claims

1. Method for performing route planning for a vehicle (1) that comprises an energy store (2) for storing the energy for driving the vehicle (1), in which
- appointment data, which comprise geographical positions of destinations on a route to be planned and associated time data, are transmitted to a computation unit (7) that is coupled to a data memory (6) that stores data pertaining to a route network for the vehicle (1) and data pertaining to the geographical positions of power supply devices,
- the computation unit (7) checks whether a route sequence is computable that links the geographical positions of the destinations associated with the appointment data such that the destinations are reached for the associated time data of the appointment data,
- if the computation unit (7) cannot be used to compute such a route sequence, it determines adapted appointment data for which such a route sequence is computable, and
- the adapted appointment data are output,
**characterized**
- **in that** the check to determine whether the route sequence is computable determines and takes into consideration the forecast remaining amount of energy in the energy store (2) of the vehicle (1) for driving through the route sequence, and
- **in that** the computation unit (7), on computing the route sequence, optimizes the time that is useable by the user of the vehicle (1), taking into consideration that the amount of energy in the energy store (2) of the vehicle (1) can be increased by a power supply device during a stationary time for the vehicle (1) during an appointment of the user.

2. Method according to Claim 1, **characterized in that** the adapted appointment data involve new time data being associated with geographical positions of destinations.

3. Method according to Claim 1 or 2, **characterized in that** the adapted appointment data have destinations deleted.

4. Method according to one of the preceding claims, **characterized in that** the computation unit (7) computes the forecast remaining amount of energy in the energy store (2) on the basis of the forecast energy consumption for driving through the route sequence, wherein the forecast energy consumption for driving the vehicle (1) and the forecast energy consumption of internal loads of the vehicle (1) are taken into consideration.

5. Method according to one of the preceding claims, **characterized in that**
- the data memory (6) further stores data pertaining to geographical positions of stationary locations, which include parking spaces or power supply devices, for the vehicle (1) for the destinations in the destination sequence,
- the computation unit (7), on computing a route sequence, further ascertains, for the destinations in the destination sequence, stationary locations associated with the respective destinations close to the respective destination, determines an associated stationary location for each destination in the destination sequence, said determination taking into consideration the distance of the stationary location from the associated destination, the geographical position of the next destination or the geographical positions of the stationary locations at the next destination and/or the forecast remaining amount of energy in the energy store (2) for driving through the route sequence, and the route sequence is compiled from routes between stationary locations at successive destinations in the destination sequence, and
- the check to determine whether a route sequence links the geographical positions of the destinations associated with the appointment data such that the destinations are reached for the associated time data of the appointment data takes into consideration a period that a user needs in order to get from the stationary location that is associated with the destination to this destination.

6. Method according to Claim 5, **characterized in that** while the computed route sequence is driven through, the remaining amount of energy in the energy store (2) of the vehicle (1) is sensed and is compared with the forecast remaining amount of energy in the vehicle (1) for a corresponding route position, and, if the discrepancy between the sensed remaining amount of energy and the forecast remaining amount of energy exceeds a limit value, then a check is performed to determine whether the destinations in the route sequence will still be reached for the associated time data of the appointment data, the forecast remaining amount of energy in the energy store (2) of the vehicle (1) being determined for driving through the route sequence.

7. Method according to Claim 6 or 7, **characterized in that** if the result of the check is that the destinations on the routes cannot be reached for the associated time data, then an adapted route sequence is computed or adapted appointment data are determined and the adapted route sequence or the adapted appointment data are output.

8. Apparatus for route planning for a vehicle (1) that comprises an energy store (2) for storing the energy for driving the vehicle (1), having
- a computation unit (7),
- a data memory (6), coupled to the computation unit (7), that stores data pertaining to a route network and data pertaining to geographical positions of power supply devices for the vehicle (1),
- an interface (21), coupled to the computation unit (7), that can be used to transmit appointment data, which comprise geographical positions of destinations on the route to be planned and associated time data, to the computation unit (7),
- an output unit (5), coupled to the computation unit (7), that can be used to output a route sequence computed by the computation unit (7) and/or adapted appointment data and that can be used to check whether a route sequence is computable that links the geographical positions of the destinations associated with the appointment data such that the destinations are reached for the associated time data of the appointment data, and, if no such route sequence is computable, the computation unit (7) can be used to determine adapted appointment data for which such a route sequence is computable,
**characterized in that**
- the computation unit (7) is used to determine and take into consideration the forecast remaining amount of energy in the energy store (2) of the vehicle (1) for driving through the route sequence and, on computing the route sequence, can be used to optimize the time that is useable by the user of the vehicle (1), taking into consideration that the amount of energy in the energy store (2) of the vehicle (1) can be increased by a power supply device during a stationary time for the vehicle (1) during an appointment of the user.

9. Apparatus according to Claim 8, **characterized in that**
- the apparatus has a vehicle-external module (3) and a vehicle-internal module, wherein the vehicle -external module (3) comprises the computation unit (7),
- the vehicle-external (3) and vehicle-internal modules are at least intermittently coupled to one another for data purposes via an interface (8, 11), so that at least one route sequence is transmittable from the vehicle-external module (3) to the vehicle-internal module,
- the vehicle-internal module comprises a further computation unit (12), a further output unit (13), a memory (14) for storing a route sequence transmitted by the vehicle-external module (3) and a sensor (15) for sensing the remaining amount of energy in the energy store (2) of the vehicle (1),
- the further computation unit (12) can be used, while the computed route sequence is driven through, to compare the sensed remaining amount of energy in the energy store (2) of the vehicle (1) with the forecast remaining amount of energy in the vehicle (1) for a corresponding route position in the route sequence stored in the memory (14), and, if the discrepancy between the sensed remaining amount of energy and the forecast remaining amount of energy exceeds a limit value, then the further computation unit (12) can be used to check whether the destinations in the route sequence will still be reached for the associated time data of the appointment data, the forecast remaining amount of energy in the energy store (2) of the vehicle (1) being determined for driving through the route sequence, and,
- if the result of the check is that the destinations in the route sequence cannot be reached for the associated time data, then the further computation unit (12) computes an adapted route sequence or determines adapted appointment data for output via the further output unit (13).

## Revendications

1. Procédé pour effectuer un calcul d'itinéraire pour un véhicule (1) équipé d'un accumulateur d'énergie (2) destiné à stocker de l'énergie servant à la propulsion du véhicule (1), dans lequel
- des données de destination qui comprennent des positions géographiques de lieux-cibles de l'itinéraire à planifier et des données temporelles correspondantes, sont transmises à une unité de calcul (7) qui est couplée à une mémoire de données (6) dans laquelle sont stockées des données concernant un réseau routier pour le véhicule (1) et des données relatives aux positions géographiques de dispositifs d'alimentation en énergie,
- l'unité de calcul (7) vérifie s'il est possible de calculer une séquence d'itinéraire qui relie les positions géographiques des lieux cibles liés aux données de destination de manière à atteindre les lieux cibles pour les données temporelles correspondantes des données de destination,
- dans le cas où il n'est pas possible de calculer une telle séquence d'itinéraire au moyen de l'unité de calcul (7), les données de destination adaptées pour lesquelles une telle séquence d'itinéraire peut être calculée sont déterminées, et
- les données de destination adaptées sont délivrées en sortie,
**caractérisé**
- **en ce que**, lorsqu'il est vérifié s'il est possible de calculer la séquence d'itinéraire, la quantité d'énergie résiduelle prévue se trouvant dans l'accumulateur d'énergie (2) du véhicule (1) pour parcourir la séquence d'itinéraire est déterminée et prise en compte, et
- **en ce que** l'unité de calcul (7), lors du calcul de la séquence d'itinéraire, optimise le temps utilisable pour l'utilisateur du véhicule (1), dans lequel il est tenu compte du fait que la quantité d'énergie se trouvant dans l'accumulateur d'énergie (2) du véhicule (1) peut être augmentée par un dispositif d'alimentation en énergie pendant un temps d'arrêt du véhicule (1) au cours d'une étape de l'utilisateur.

2. Procédé selon la revendication 1, **caractérisé en ce que**, lorsque les données d'étapes sont adaptées, de nouvelles données temporelles sont associées à des positions de lieux cibles.

3. Procédé selon la revendication 1 ou 2, **caractérisé en ce que**, lorsque les données d'étapes sont adaptées, des lieux cibles sont supprimés.

4. Procédé selon l'une quelconque des revendications précédentes, **caractérisé en ce que** l'unité de calcul (7) calcule la quantité d'énergie résiduelle prévue se trouvant dans l'accumulateur d'énergie (2) sur la base de la consommation d'énergie prévue pour le parcours de la séquence d'itinéraire, dans lequel la consommation d'énergie prévue pour la propulsion du véhicule (1) et la consommation d'énergie prévue de dispositifs utilisateurs internes au véhicule (1) sont prises en compte.

5. Procédé selon l'une quelconque des revendications précédentes, **caractérisé en ce que**
- des données relatives à des positions géographiques d'emplacements d'arrêt qui comprennent des emplacements de stationnement ou des dispositifs d'alimentation en énergie, sont stockées dans la mémoire des données (6) pour le véhicule (1) pour les lieux cibles de la séquence de lieux cibles,
- l'unité de calcul (7), lors du calcul d'une séquence d'itinéraire, détermine en outre pour les lieux cibles, la séquence de lieux cibles respective relative aux emplacements d'arrêt associés aux lieux cibles à proximité du lieu cible respectif, détermine pour chaque lieu cible de la séquence de lieux cibles un emplacement d'arrêt associé, dans lequel, lors de la détermination, la distance de l'emplacement d'arrêt par rapport au lieu cible correspondant, la position géographique du lieu cible le plus proche, ou les positions géographiques des emplacements d'arrêt du lieu cible le plus proche et/ou la quantité d'énergie résiduelle prévue se trouvant dans l'accumulateur d'énergie (2) pour parcourir la séquence d'itinéraire sont prises en compte, et la séquence d'itinéraire se compose d'itinéraires entre des emplacements d'arrêt de lieux cibles consécutifs de la séquence de lieux cibles, et
- lorsqu'il est vérifié si une séquence d'itinéraire relie les positions géographiques des lieux cibles correspondant aux données d'étapes, il est tenu compte du fait que les lieux cibles, pour les données temporelles correspondantes des données d'étapes ont été atteints, une durée qui est nécessaire pour qu'un utilisateur se rende de l'emplacement d'arrêt correspondant à un lieu cible jusqu'audit lieu cible est prise en compte.

6. Procédé selon la revendication 5, **caractérisé en ce que**, pendant que la séquence itinéraire calculé est parcourue, la quantité d'énergie résiduelle se trouvant dans l'accumulateur d'énergie (2) du véhicule (1) est détectée et comparée à la quantité d'énergie résiduelle prévue du véhicule (1) pour une position d'itinéraire correspondante et, dans le cas où l'écart entre la quantité d'énergie résiduelle détectée et la quantité d'énergie résiduelle prévue dépasse une valeur limite, il est vérifié si les lieux cibles de la séquence d'itinéraire, pour les données temporelles correspondantes des données d'étapes ont en outre été atteints, dans lequel la quantité d'énergie résiduelle prévue se trouvant dans l'accumulateur d'énergie (2) du véhicule (1) est déterminée pour le parcours de la séquence d'itinéraire.

7. Procédé selon la revendication 6 ou 7, **caractérisé en ce que**, dans le cas où la vérification indique que les lieux cibles des itinéraires n'ont pas pu être atteints pour les données temporelles correspondantes, une séquence d'itinéraire adaptée est calculée ou des données d'étapes adaptées sont déterminées et la séquence d'itinéraire adaptée ou les données d'étapes adaptées sont délivrées en sortie.

8. Dispositif de calcul d'itinéraire pour un véhicule (1) équipé d'un accumulateur d'énergie (2) destiné à stocker de l'énergie servant à la propulsion du véhicule (1), comportant
- une unité de calcul (7),
- une mémoire de données (6) couplée à l'unité de calcul (7), dans laquelle sont stockées des données concernant le réseau routier et des données relatives à des positions géographiques de dispositifs d'alimentation en énergie pour le véhicule (1),
- une interface (21) couplée à l'unité de calcul (7), interface par l'intermédiaire de laquelle les données d'étapes, qui comprennent des positions géographiques de lieux cibles de l'itinéraire à planifier et des données temporelles correspondantes, peuvent être transmises à l'unité de calcul (7),
- une unité de sortie (5) couplée à l'unité de calcul (7), unité de sortie au moyen de laquelle une séquence d'itinéraire calculée par l'unité de calcul (7) et/ou des données d'étapes adaptées peuvent être délivrées en sortie et au moyen de laquelle on peut vérifier s'il est possible de calculer une séquence d'itinéraire qui relie les positions géographiques des lieux cibles correspondant aux données d'étapes de manière à atteindre les lieux cibles pour les données temporelles correspondantes des données d'étapes et, dans le cas où il est impossible de calculer une telle séquence d'itinéraire, des données d'étapes adaptées peuvent être déterminées au moyen de l'unité de calcul (7), données pour lesquelles il est possible de calculer une telle séquence d'itinéraire,
**caractérisé en ce que**,
- au moyen de l'unité de calcul (7), la quantité d'énergie résiduelle prévue se trouvant dans l'accumulateur d'énergie (2) du véhicule (1) est prise en compte pour le parcours de la séquence d'itinéraire et, lors du calcul de la séquence d'itinéraire, le temps utilisable pour l'utilisateur du véhicule (1) peut être optimisé, dans lequel on prend en compte le fait que la quantité d'énergie se trouvant dans l'accumulateur d'énergie (2) du véhicule (1) peut être augmentée par un dispositif d'alimentation en énergie pendant un temps d'arrêt du véhicule (1) au cours d'une étape de l'utilisateur.

9. Dispositif selon la revendication 8, **caractérisé en ce que**
- le dispositif comporte un module externe au véhicule (3) et un module interne au véhicule, dans lequel le module externe au véhicule (3) comprend l'unité de calcul (7),
- le module externe au véhicule (3) et le module interne au véhicule sont couplés l'un à l'autre au moins par instants par une technique d'échange de données par l'intermédiaire d'une interface (8, 11) de manière à ce qu'au moins une séquence d'itinéraire puisse être transmise du module externe au véhicule (3) au module interne au véhicule,
- **en ce que** le module interne au véhicule comprend une autre unité de calcul (12), une autre unité de sortie (13), une mémoire (14) pour le stockage d'une séquence d'itinéraire transmise par le module externe au véhicule (3) et un capteur (15) pour la détection de la quantité d'énergie résiduelle se trouvant dans l'accumulateur d'énergie (2) du véhicule (1),
- au moyen de l'autre unité de calcul (12), pendant le parcours de la séquence d'itinéraire calculée, la quantité d'énergie résiduelle se trouvant dans l'accumulateur d'énergie (2) du véhicule (1) peut être comparée à la quantité d'énergie résiduelle prévue du véhicule (1) pour une position d'itinéraire correspondante de la séquence d'itinéraire stockée dans la mémoire (14) et, dans le cas où l'écart entre la quantité d'énergie résiduelle détectée et la quantité d'énergie résiduelle prévue dépasse une valeur limite, il est possible de vérifier, au moyen de l'autre unité de calcul (12), si les lieux cibles de la séquence d'itinéraire, pour les données temporelles correspondantes des données d'étapes, ont en outre été atteints, dans lequel la quantité d'énergie résiduelle prévue se trouvant dans l'accumulateur d'énergie (2) du véhicule (1) pour parcourir la séquence d'itinéraire est déterminée, et
- dans le cas où la vérification indique que les lieux cibles de la séquence d'itinéraire n'ont pas pu être atteints pour les données temporelles correspondantes, l'autre unité de calcul (12) calcule une séquence d'itinéraire adaptée ou des données d'étapes adaptées destinées à être délivrées en sortie par l'intermédiaire de l'autre unité de sortie (13).
